(19) Europäisches Patentamt
European Patent Office
Office européen des brevets

(11) EP 2 700 717 B1

(12) **EUROPEAN PATENT SPECIFICATION**

(45) Date of publication and mention of the grant of the patent:
**01.06.2016 Bulletin 2016/22**

(51) Int Cl.:
***C12Q 1/25*** (2006.01) ***G01N 33/68*** (2006.01)
***C12Q 1/34*** (2006.01) ***G01N 33/58*** (2006.01)
***C07H 17/075*** (2006.01)

(21) Application number: **12005989.4**

(22) Date of filing: **22.08.2012**

(54) **Method for the diagnosis of lysosomal storage diseases**

Verfahren zur Diagnose lysomaler Speicherkrankheiten

Procédé pour le diagnostic de maladies de stockage lysosomal

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.02.2014 Bulletin 2014/09**

(73) Proprietors:
- **Przybylski, Michael**
**65468 Trebur (DE)**
- **Maeser, Stefan**
**63867 Johannesberg (DE)**

(72) Inventors:
- **Maeser, Stefan**
**63867 Jahannesberg (DE)**
- **Przybylski, Michael, Prof.**
**65468 Trebur (DE)**
- **Cozma, Claudia**
**78464 Konstanz (DE)**

(74) Representative: **Müller-Boré & Partner Patentanwälte PartG mbB**
**Friedenheimer Brücke 21**
**80639 München (DE)**

(56) References cited:
**WO-A1-2012/027612 US-A1- 2011 118 132**

- **GERBER S A ET AL: "DIRECT PROFILING OF MULTIPLE ENZYME ACTIVITIES IN HUMAN CELL LYSATES BY AFFINITY CHROMATOGRAPHY/ELECTROSPRAY IONIZATION MASS SPECTROMETRY: APPLICATION TO CLINICAL ENZYMOLOGY", ANALYTICAL CHEMISTRY, AMERICAN CHEMICAL SOCIETY, US, vol. 73, no. 8, 15 April 2001 (2001-04-15) , pages 1651-1657, XP008045697, ISSN: 0003-2700, DOI: 10.1021/AC0100650**
- **BRIAN J. WOLFE ET AL: "Tandem Mass Spectrometry for the Direct Assay of Lysosomal Enzymes in Dried Blood Spots: Application to Screening Newborns for Mucopolysaccharidosis II (Hunter Syndrome)", ANALYTICAL CHEMISTRY, vol. 83, no. 3, 1 February 2011 (2011-02-01), pages 1152-1156, XP055052397, ISSN: 0003-2700, DOI: 10.1021/ac102777s**

EP 2 700 717 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention relates to a method for the diagnosis of a lysosomal storage disease (LSD) in a subject which is based on determining the activity of lysosomal enzymes with the help of 4-alkyl umbelliferyl derivatives. The present invention further relates to said 4-alkyl umbelliferyl derivatives for use in the diagnosis of an LSD, and a kit for the diagnosis of an LSD.

Lysosomes, a kind of intracellular membrane vesicle, are responsible for the degradation and metabolism of many endogenous substances such as lipids, carbohydrates and proteins by the action of lysosomal enzymes. If, as a result of an inherited genetic defect or a biochemical defect, a lysosomal enzyme is absent or deficient regarding its function, LSDs are manifesting by several life-threatening symptoms.

LSDs are hereditary, mostly autosomal recessive metabolic disorders which can lead to severe and progressing disabilities and death. The symptoms of LSDs include mental retardation and bone, muscle and organ malformations, such as enlargement of the heart, liver and spleen, some of them being life-threatening already at early childhood. Approximately 60 to 70 different LSDs are known worldwide. However, only about a dozen of these "rare" metabolic diseases have previously been characterized biochemically and are currently amenable to therapy, e.g. by replacement with the specific absent or deficient enzyme (Enzyme Replacement Therapy; ERT). Well-known examples of LSDs are e.g. Gaucher's Disease, Niemann-Pick-Disease, various Mucopolysaccharidoses (MPSs), various Mucolipidoses (MLs), and other metabolic diseases. Previously estimated frequencies of LSDs are in the range of about 1 out of 7500 births. Table 1 shows various LSDs and the affected lysosomal enzymes.

Table 1: Various LSDs and the respective affected lysosomal enzymes

| **Lysosomal storage disease** | **affected enzyme** |
|---|---|
| Group 1: Type II Glycogen storage diseases | |
| Pompe disease | α-Glucosidase |
| Group 2: Mucopolysaccharidoses (MPSs) | |
| MPS-I | α-L-Iduronidase |
| MPS-II | Iduronate sulfatase |
| MPS-IIIA | Heparan sulfamidase |
| MPS-IIIB | N-acetylglucosaminidase |
| MPS-IIIC | Acetyl-CoA:α-glucosaminide acetyltransferase |
| MPS-IIID | N-acetylglucosamine-6-sulfatase |
| MPS-IVA | Galactose-6-sulfate sulfatase |
| MPS-IVB | β-Galactosidase |
| MPS-VI | N-acetylgalactosam ine-4-sulfatase |
| MPS-VI | β-Glucuronidase |
| Group 3: Mucolipidoses (MLs) | |
| ML-I | Sialidase |
| ML-I | N-acetylglucosamine-1-phosphotransferase |
| ML-III | N-acetylglucosamine-1-phosphotransferase |
| Group 4: Oligosaccharidoses | |
| Schindler Disease/Kanzaki Disease | α-N-Acetylgalactosaminidase |
| α-Mannosidosis | α-D-Mannosidase |
| β-Mannosidosis | β-Mannosidase |
| α-Fucosidosis | α-L-Fucosidase |
| Aspartylglucosaminuria | Aspartylglucosaminase |
| Group 5: Lipidoses | |

(continued)

| Lysosomal storage disease | affected enzyme |
|---|---|
| Niemann-Pick Disease Type C | Cholesterol-O-Acetyltransferase |
| Niemann-Pick Disease Type D | Neutral sphingomyelinase |
| Neuronal ceroid lipofuscinoses | Lysosomal protease(s), Tripeptidylpeptidase I |
| Wolman Disease | Acid lipase |
| Acid lipase disease | Lipase |
| Group 6: Sphingolipidoses | |
| Fabry's Disease | α-Galactosidase |
| Gaucher's Disease | β-Glucocerebrosidase |
| Niemann-Pick-Disease Type A | Acid sphingomyelinase |
| Niemann-Pick-Disease Type B | Acid sphingomyelinase |
| Krabbe's Disease | β-Galactosylceramidase |
| GM1 gangliosidosis | β-Galactosidase |
| Tay-Sachs Disease | Hexosaminidase A |
| Sandhoff Disease | Hexosaminidase A and B |
| Metachromatic Leukodystrophy | Arylsulfatase A |
| Farber Disease | Ceramidase |
| Multiple sulfatase deficiency | Sulfatase-modifying factor-1 |
| Galactosialidosis | Cathepsin A |

In some of these cases, ERTs have been developed and are clinically employed. However, these have been shown to be curative only in some cases, and mostly rather just slow down or arrest progression of the disease. Further, therapies at later stages of the diseases are often no longer effective. Accordingly, it is of vital importance to start the treatment as soon as possible. However, this requires early and specific diagnostic methods.

So far, the diagnosis of LSDs still relies mostly on invasive methods, wherein so called storage cells are detected by staining techniques in e.g. skin, liver, spleen or bone marrow material obtained from biopsies. However, these methods require a great amount of expertise on the side of the examining pathologist, since not only storage cells occurring in patients suffering from an LSD, but also so called pseudo storage cells, which can also occur in subjects not suffering from an LSD, can be detected. Moreover, a reliable diagnosis of a particular LSD with the help of storage cells is very difficult and prone to errors, misdiagnosis and, as a consequence, wrong treatments. Further, measuring certain metabolites in urine or blood samples can provide hints at the presence of an LSD. However, a reliable diagnosis of a particular LSD is still not possible.

In this context, WO 2012/027612 A1 describes substrates, internal standards, enzymatic assays and screening methods for the analysis of various LSDs. Further, Gerber et al. (Gerber, S. A. et al.; Anal. Chem., 73; 2001; pp. 1651-1657) describes the diagnosis of Sanfilippo syndrome using enzyme analysis by affinity capture followed by mass spectrometry. Moreover, Wolfe et al. (Wolfe, B. J. et al.; Anal. Chem., 83; 2011; pp. 1152-1156) describes the diagnosis of Hunter syndrome in dried blood spots using tandem mass spectrometry. Finally, US 2011/0118132 A1 describes enzymatic assays using umbelliferone substrates.

As far as it is available, the gold standard in the diagnosis of LSDs today is the determination of the catalytic activity of affected lysosomal enzymes. This is mostly done by fluorimetric methods which allow a reliable diagnosis of particular LSDs. However, a fundamental drawback of these methods is the necessity to determine the activity of each relevant lysosomal enzyme one at a time. Since current fluorimetric methods mostly require the use of fresh blood samples, and said methods cannot be easily automated, the normal procedure is often to look for one specific LSD and, in case the result is negative, to look for another specific LSD and so on, until one particular LSD can be diagnosed. This procedure considerably prolongs the time until a reliable diagnosis can be established. Moreover, fluorimetric methods of diagnosis are only available for a limited range of known LSDs.

Accordingly, the technical problem underlying the present invention is to provide methods for the simple, fast,

and accurate diagnosis of LSDs. Such methods should show a high sensitivity, need a minimal amount of patient sample material, and provide the ability to determine the activity of more than one lysosomal enzyme at the same time. Further, such methods should allow the actual determination to be effected by fluorimetry as well as mass spectrometry, since mass spectrometry, being extremely sensitive but also complex and expensive, could be used to validate fluorimetric methods which are more simple, economic and widespread.

The solution to the above technical problem is achieved by the embodiments characterized in the claims.

In particular, in a first aspect the present invention relates to a method for the in vitro diagnosis of a lysosomal storage disease (LSD) in a subject, comprising the steps of:

(a) providing a blood sample of the subject;

(b) incubating the blood sample in the presence of more than one 4-alkyl umbelliferyl derivatives according to formula (1):

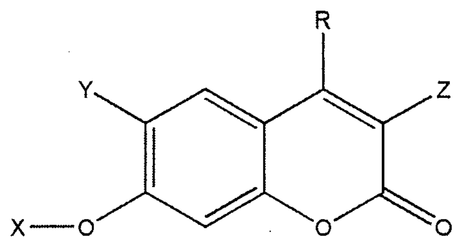

wherein:

| | |
|---|---|
| R | is selected from the group consisting of H, methyl, ethyl, propyl, isopropyl, tert.-butyl, pentyl, hexyl, and octyl; |
| X | is sulfate or a sugar moiety that is specifically recognized by a lysosomal enzyme, deficiency of which is the cause of an LSD; and |
| Y and Z | are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15; |

and wherein said 4-alkyl umbelliferyl derivatives all differ from each other in both substituents R and X, but are identical in both substituents Y and Z;

(c) determining the amount of more than one 4-alkyl umbelliferones corresponding in the substituents R, Y, and Z to the 4-alkyl umbelliferyl derivative used in step (b) in the sample by mass spectrometry; and

(d) assessing the activity of the more than one lysosomal enzymes specifically recognizing the sulfate or sugar moiety X based on the amounts of 4-alkyl umbelliferone determined in step (c).

The method of the present invention is based on the specific cleavage of 4-alkyl umbelliferyl derivatives by lysosomal enzymes. One product of this cleavage is 4-alkyl umbelliferone which is not an endogenous blood compound and is not further processed by lysosomal or other enzymes. Accordingly, it remains in solution after the enzymatic reaction and can be detected by fluorimetry or mass spectrometry (Fig. 1).

The LSDs that can be determined with the method of the present invention are not particularly limited, provided that the affected lysosomal enzyme and its substrate are known. In particular, the LSD that can be determined can be selected from the group consisting of Type II Glycogen Storage Diseases, Mucopolysaccharidoses (MPSs), Mucolipidoses (MLs), Oligosaccharidoses, Lipidoses, and Sphingolipidoses. An example for a Type II Glycogen Storage Disease that can be determined with the method of the present invention is Pompe Disease. Examples for MPSs that can be determined with the method of the present invention include Hurler syndrome (Mucopolysaccharidosis type I; MPS-1), Hunter syndrome (MPS-II), Sanfilippo syndrome A (MPS-IIIA), Sanfilippo syndrome B (MPS-IIIB), Sanfilippo syndrome C (MPS-IIIC), Sanfilippo syndrome D (MPS-IIID), classic Morquio syndrome (MPS-IVA), Morquio B Disease (MPS-IVB), Maroteaux-Lamy syndrome (MPS-VI), and Sly syndrome (MPS-VII). Examples for MLs that can be determined with the method of the present invention include Sialidosis (Mucolipidosis type I; ML-I), I-cell Disease (ML-II), and Pseudo-Hurler Polydystrophy (ML-III). Examples for Oligosaccharidoses that can be determined with the method of the present invention include Schindler Disease/Kanzaki Disease, α-Mannosidosis, β-Mannosidosis, α-Fucosidosis, and Aspartylglucosaminuria. Examples for Lipidoses that can be determined with the method of the present invention include Niemann-Pick Disease Type C, Niemann-Pick Disease Type D, Neuronal ceroid lipofuscinoses, Wolman Disease, and Acid lipase disease. Examples for Sphingolipidoses that can be determined with the method of the present invention include Fabry's Disease, Niemann-Pick-Disease Type A, Niemann-Pick Disease Type B, Gaucher's Disease, Krabbe's Disease, GM1

Gangliosidosis, Tay-Sachs Disease, Sandhoff Disease, Metachromatic Leukodystrophy, Farber Disease, Multiple sulfatase deficiency, and Galactosialidosis.

In preferred embodiments of the present invention, the subject for which a diagnosis should be established is a human.

The blood sample to be used in the method of the present invention can be any form of blood sample, e.g. whole blood, serum, plasma or dried blood spots (DBSs). Since DBSs only require a minimal amount of blood from the subject (about 100 µl fresh or EDTA-treated blood per spot), and can easily be stored at room temperature for a considerable amount of time, DBSs are particularly preferred.

Incubation as defined in step (b) of the method of the present invention is preferably incubation for 12 to 48 hours at a temperature of about 37°C. However, a person skilled in the art will know how this incubation can be modified, e.g. by incubating for a longer time at a lower temperature, or by incubating for a shorter time at a higher temperature, provided that said temperatures allow the proper functioning of the respective enzymes. Further, the exact duration of incubation can differ between different enzymes to be determined. Preferably, incubation is done in an aqueous medium.

The 4-alkyl umbelliferone derivative to be used in the method of the present Invention is as defined above. Said derivatives are sometimes referred to herein as "substrate", e.g. in connection with the specific LSD that is connected to deficiency of the lysosomal enzyme that recognizes a particular derivative. As an example, the derivative 4-alkyl-umbelliferyl-α-1-iduronide, which is recognized by α-L-iduronidase, deficiency of which is the cause for MPS-I, can be referred to as "MPS-1 substrate" herein.

The substituent R of the 4-alkyl umbelliferone derivative as defined above is selected from the group consisting of methyl, ethyl, propyl, isopropyl, tert.-butyl, pentyl, hexyl, and octyl. The alkyl as defined above can be substituted. The substituent X of the 4-alkyl umbelliferone derivative as defined above can be sulfate, which is specifically recognized by e.g. N- acetylgalactosamine-4-sulfatase, deficiency of which is the cause for MPS-VI, or a sugar moiety that is specifically recognized by a lysosomal enzyme, deficiency of which is the cause of an LSD. The term "sugar moiety" as used herein relates to any carbohydrate or carbohydrate derivative that satisfies the above prerequisite of being recognized by a lysosomal enzyme, deficiency of which is the cause of an LSD.

Respective sugar moieties are known in the art. In particular, a person skilled in the art will know what enzyme is related to a particular LSD, and will further know the substrate of said enzyme and, thus, what the substituent X should be. The substituents Y and Z of the 4-alkyl umbelliferone derivative as defined above can be independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15.

In particular, LSDs and the corresponding substituent X as defined above can be Pompe disease and α-glucose; MPS-I and α-1-iduronide; MPS-II and α-iduronate-2-sulfate; MPS-IIIA and 2-sulfamino-2-deoxy-α-D-glucopyranoside; MPS-IIIB and 2-acetamido-2-deoxy-α-D-glucopyranoside; MPS-IIIC and α-D-2-amino-glucopyranoside; MPS-IIID and 2-acetamido-2-deoxy-6-sulfate-ß-D-glucopyranoside; MPS-IVA and ß-D-galactopyranoside-6-sulfate sodium salt; MPS-IVB and ß-D-galactopyranoside; MPS-VI and sulfate; MPS-VII and ß-D-glucuronide; ML-I and α-D-N-acetyl neuraminic acid; ML-II and one of ß-D-glucuronide, N-acetyl-glucosamine, ß-D-galactoside, and α-L-fucoside; ML-III and α-D-manno-pyranoside; Schindler Disease/Kanzaki Disease and α-N-acetylgalactosamine; α-Mannosidosis and α-D-mannose; β-Mannosidosis and β-mannose; α-Fucosidosis and α-L-fucose; Aspartylglucosaminuria and aspartylglucosamine; Niemann-Pick Disease Type C and cholesterol; Niemann-Pick Disease Type D and shingolipids; Neuronal ceroid lipofuscinoses and the amino-terminal tripeptidyl derivative GFFLAF; Wolman Disease and short chain fatty-acyl esters such as palmitate, eloidate, lignocerate and cholesteryl esters; Fabry's Disease and α-D-galactopyranoside; Gaucher's Disease and β-D-glucopyranoside; Niemann-Pick Disease Type A and phosphocholine; Niemann-Pick Disease Type B and phosphocholine; Krabbe's Disease and β-D-galactopyranoside; GM1-Gangliosidosis and β-D-galactopyranoside; Tay-Sachs Disease and α-N-acetylglucosamine; Sandhoff Disease and β-N-acetylglucosamine; Metachromatic Leukodystrophy and sulfate; Farber Disease and ceramide; Multiple sulfatase deficiency and sulfate; and Galactosialidosis and N-acetylneuraminic acid or β-galactose. In all of the above LSD - substituent X combinations, the substituents Y and Z can be as defined above. Figure 2 shows examples of various LSDs, the affected enzymes, and respective substrates.

Step (c) of the method of the present invention, i.e. determining the amount of more than one 4-alkyl umbelliferones corresponding in the substituent R to the 4-alkyl umbelliferyl derivative used in step (b) of the method of the present invention in the sample, is effected by mass spectrometry. In this context, the expression "4-alkyl umbelliferone corresponding in the substituent R to the 4-alkyl umbelliferyl derivative used in step (b)" as used herein refers to the fact that said 4-alkyl umbelliferone and said 4-alkyl umbelliferyl derivative have the same substituent R, since said 4-alkyl umbelliferone is the product of the cleavage of said 4-alkyl umbelliferyl derivative by the respective lysosomal enzyme.

It is a notable feature of the method of the present invention that the above step can also be effected by fluorimetry and mass spectrometry in parallel. Since in both cases the same substrates are used, and the actual execution of the method of the present invention is largely identical up to step (c) (cf. Examples 1 and 2), results of either method can be easily compared and correlated to results of the other. Accordingly, the method of the present invention can e.g. be thoroughly validated by mass spectrometric determination, which is extremely sensitive, but also quite complex, costly and laborious, and the results obtained can be correlated to results obtained by fluorimetric determination, which

is more simple, inexpensive and widespread, allowing e.g. the use of a thoroughly validated method also for laboratories that do not have access to mass spectrometry. In particular, the mass spectrometric method allows molecular determination with absolute specificity for each disease; further, it can be used for multiplex determinations. The fluorimetric method is the method most widely used in e.g. hospitals. It is more simple, and less expensive than mass spectrometry. Once a LSD has been identified unequivocally, the fluorimetric determination is regarded as the "gold standard".

In the case of fluorimetric determination, a calibration curve is established using the respective 4-alkyl umbelliferone in known concentrations as standards. Fluorimetric determination can be done with any fluorimeter equipped with a specific umbelliferone filter. The excitation and emission wavelength of 4-alkyl umbelliferone are 360 and 400 nm, respectively. Suitable methods for the fluorimetric determination of 4-alkyl umbelliferone are known in the art.

In the case of mass spectrometric determination, a suitable internal standard has to be used. Respective standards are known in the art. In a preferred embodiment, 4-alkyl-umbelliferone-1,2,3,4-$^{13}$C is used, wherein the alkyl is preferably methyl. Parameters for mass spectrometric determinations are specific for different types of mass spectrometers, e.g. ion trap or triple-quadruple mass spectrometers, which can both be used in the method of the present invention, and for the type of measurement, e.g. single reaction monitoring mass spectrometry (SRM-MS) or multiple reaction monitoring mass spectrometry (MRM-MS) which both can be used in the method of the present invention. In a particular embodiment, MRM-MS is used. For all mass spectrometric assays, no additional sample cleanup is required. Accordingly, the incubation solution is mixed with the internal standard and directly injected into the electrospray source of the mass spectrometer. Suitable methods for the mass spectrometric determination of 4-alkyl umbelliferone are known in the art.

According to the present invention, detection in step (c) is detection by mass spectrometry and

(i) more than one 4-alkyl umbelliferyl derivative according to formula (I) is used in step (b), wherein said 4-alkyl umbelliferyl derivatives all differ from each other in both substituents R and X, but are identical in both substituents Y and Z,
(ii) more than one corresponding 4-alkyl umbelliferone is detected in step (c), and
(iii) the activity of more than one lysosomal enzyme specifically recognizing the different substituents X is assessed in step (d).

In this "multiplex" mode of the method of the present invention, the activity of more than one lysosomal enzyme can be determined in one sample, satisfying the long-felt need to screen for more than one LSD at the same time and in the same sample. This is facilitated by the use of different 4-alkyl umbelliferyl derivatives as defined above which all differ from each other in both substituents R and X. In this manner, substrates for a multitude of different LSDs can be employed. Cleavage of these substrates by the respective lysosomal enzymes leads to the generation to 4-alkyl umbelliferones, which for each LSD differ in the alkyl residue R, thus enabling the discrimination and measurement of said 4-alkyl umbelliferones by mass spectrometry.

In the method of the present invention, the activity of lysosomal enzymes, deficiency of which is the cause for various LSDs, can be determined. In case the activity of a particular enzyme is determined to be 20% lower than the average activity of said enzyme in healthy control samples, the corresponding LSD is diagnosed.

The method of the present invention will enable a substantial extension and improvement of previously employed LSD diagnostics, and will lead to a rapid, reliable and widely applicable spectrum of clinical diagnostics for said diseases. A major advantage of the present invention is the common substrates both for fluorimetric and mass spectrometric determination of enzyme activity. This feature enables the easy correlation of data obtained e.g. by different laboratories, regardless of the fact which of the two determination methods have been used. Further, the method of the present invention is able to distinguish between healthy subjects, (heterozygous) carriers of the disease, and (homozygous) subjects suffering from the disease.

In another aspect, the present invention relates to a 4-alkyl umbelliferyl derivative according to formula (I):

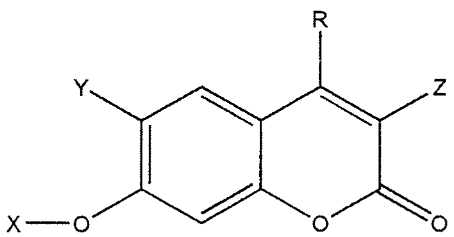

wherein:

R is selected from the group consisting of H, methyl, ethyl, propyl, isopropyl, tert.-butyl, pentyl, hexyl, and octyl;
X is sulfate or a sugar moiety that is specifically recognized by a lysosomal enzyme, deficiency of which is the cause of an LSD; and
Y and Z are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15;

for use in the diagnosis of an LSD.

In a preferred embodiment, diagnosis of an LSD is accomplished using the method of the present invention. In this aspect of the present invention, the 4-alkyl umbelliferyl derivative, the substituents R, X, Y, and Z, the alkyl, the sugar moiety and the LSD are as defined above for the method of the present invention. In a further aspect, the present invention relates to the use of a 4-alkyl umbelliferyl derivative according to formula (I):

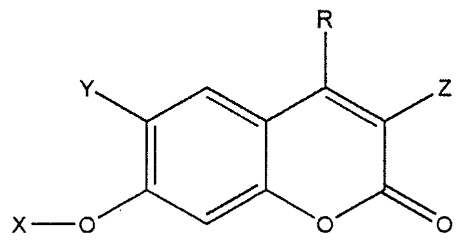

wherein:

R is selected from the group consisting of H, methyl, ethyl, propyl, isopropyl, tert.-butyl, pentyl, hexyl, and octyl;
X is sulfate or a sugar moiety that is specifically recognized by a lysosomal enzyme, deficiency of which is the cause of an LSD; and
Y and Z are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15;

in the in vitro diagnosis of an LSD.

In a preferred embodiment, diagnosis of an LSD is accomplished using the method of the present invention. Also in this aspect of the present invention, the 4-alkyl umbelliferyl derivative, the substituents R, X, Y, and Z, the alkyl, the sugar moiety and the LSD are as defined above for the method of the present invention.

In a final aspect, the present invention relates to a kit for the diagnosis of a lysosomal storage disease (LSD) in a subject, comprising at least one of the 4-a!kyl umbelliferyl derivatives as defined above.

In a preferred embodiment, the kit of the present invention further comprises 4-alkyl umbelliferones that can be used as standards for the fluorimetric and/or mass spectrometric determination as defined above for the method of the present invention. Moreover, the kit of the present invention can further comprise suitable buffers, solutions, reagents and disposables.

The figures show:

Figure 1: General scheme for lysosomal enzyme activity determination - principle of the method of the present invention

The specific substrate of a lysosomal enzyme is a compound formed by a sugar moiety or sulfate recognized by a certain enzyme (for example α-galactose for α-galactosidase, α-iduronate for α-iduronase, sulfate for aryl-sulfatase, etc.) with an umbelliferyl moiety. This umbelliferyl moiety is not an endogenous compound for blood and is not recognized and processed by the lysosomal enzymes; it is remaining in solution after the enzymatic reaction and can be quantified by fluorimetry and/or by mass spectrometry. By changing the alkyl rest on the umbelliferyl moiety (R) with hydrogen, methyl, ethyl, propyl etc., umbelliferyl derivatives are formed with different mass spectrometric characteristics. Using a mixture of different substrates with different umbelliferyl residues in the same experiment and by adding an internal standard, there is the possibility of multiplexing several enzyme determinations.

Figure 2: Summary of the characteristic sugar moieties recognized and processed by lysosomal enzymes in clinical diagnostics

A collection of different substrates used for diagnosis of lysosomal disorders, as for example: 4-alkyl-umbelliferyl-α-1-iduronide for the diagnosis of MPS-I; 4-alkyl-umbelliferyl-α-iduronate-2-sulphate for MPS-II; 4-alkyl-umbelliferyl-2-acetamido-2-deoxy-α-D-glucopyranoside for MPS-IIIB, 4-alkyl-umbelliferyl-β-D-galactopyranoside-6-sulfate sodium salt for MPS-IVA. For multiplexing 2, 3 or more enzyme determinations, the alkyl substituent is different (e.g. H, $CH_3$, $CH_2$-$CH_3$- etc.) to quantify by multiple-reaction-monitoring mass-spectrometry (MRM-MS) in the presence

of an internal standard (4-methylumbelliferone-1,2,3,4-$^{13}$C).

Figure 3: Fluorimetric determination of enzymatic activity

Dried blood spots (DBSs) obtained by cutting 3 mm spots from the Protein Saver Card with dried blood are placed into a 96-wells black plate in duplicates. Substrate solution is added and the plate is agitated at 20°C for 45 minutes. The pH is changed to 4.5 and the plate is incubated at 37°C for 48h. The product obtained is an umbelliferone derivative with fluorescence characteristics of excitation at 360 nm and emission at 400 nm. Quantification is carried out using an external standard curve (using the same umbelliferyl moiety).

Figure 4: Multiple reaction monitoring mass spectrometry determination of lysosomal enzyme activity

DBSs obtained by cutting 3 mm spots from the Protein Saver Card with dried blood are placed into a 96-wells black plate in duplicates. Substrate solution is added and the plate is agitated at 20°C for 45 minutes. The pH is changed to 4.5 and the plate is incubated at 37°C for 48h. The experimental set up can be done also in Eppendorf cups. After the incubation, the reaction is quenched and the quantification is carried out by adding a solution of internal standard with known concentration and measuring by MRM-MS. Mass spectrometry offers the possibility of multiplexing assay for the lysosomal enzymes when the product of the reaction differs in mass, by substitution in position 4 of the umbelliferone and by employing isotopic variants.

Figure 5: Multiple reaction monitoring mass spectrometry determination of MPS-1

Lysosomal α-L-iduronase contained in dry blood spots cleaves during the incubation at 37°C the terminal α-L-iduronate rest with formation of 4-methyl-umbelliferone. After the reaction is quenched by changing the pH to 1.9, internal standard solution (4-methyl-umbelliferone-1,2,3,4-$^{13}$C) at 0.3 μM is added. The intensity of the signal in case A (healthy control sample) is higher for the product and the concentration is calculated at 0.9 μM/spot/48h and in case B (MPS-I patient sample) at 0.32 μM/spot/48h.

Figure 6: Multiple reaction monitoring mass spectrometry determination of MPS-II

Lysosomal iduronate sulfatase contained in dry blood spots cuts during the incubation at 37°C the sulfate on the iduronate cycle and the α-L-iduronase cuts the terminal α-L-iduronate rest with the formation of 4-methyl-umbelliferone. The determination for MPS-II must be done in parallel with MPS-I to pinpoint the deficient enzyme. After the reaction is quenched by changing the pH to 1.9, internal standard solution (4-methyl-umbelliferone-1,2,3,4-$^{13}$C) at 0.3 μM is added. The intensity of the signal in case A (healthy control sample) is higher for the product and the concentration is calculated at 0.539 μM/spot/48h and in case B (MPS-I patient sample) at 0.31 μM/spot/48h.

Figure 7: Multiple reaction monitoring mass spectrometry determination of MPS-I

Lysosomal α-galactosidase contained in dry blood spots cuts during the incubation at 37°C the terminal α-galactose rest with the formation of 4-methyl-umbelliferone. After the reaction is quenched by changing the pH to 1.9, internal standard solution (4-methyl-umbelliferone-1,2,3,4-$^{13}$C) at 0.3 μM is added. The intensity of the signal in case A (healthy control sample) is higher for the product and the concentration is calculated at 0.998 μM/spot/48h and in case B (MPS-I patient sample) at 0.31 μM/spot/48h.

Figure 8: Synthesis of MPS-I substrate

The synthesis of MPS-I substrate is a multiple step process starting with L-glucose. L-glucose is acetylated and converted into its β-isomer, which is then substituted in position 1 by chlorination. The Cl atom is replaced by an acetyl group with the conversion into the α-isomer of the sugar compound. The 1,2,3,4,6-penta-O-acetyl-alpha-L-idopyranose is condensed specifically in position 1 with an umbelliferone derivate. 4-alkyl-umbelliferone-2,3,4,6-tetra-O-acetyl-α-L-idopyranoside is then de-protected and the hydroxyl group in position 6 is oxidized in the presence of platinum oxide to obtain the MPS-I substrate.

Figure 9: Characterization of 4-methyl-umbeltiferyl-α-L-iduronate by HPLC and mass spectrometry

20 μg 4-methyl-umbelliferyl-α-L-iduronate was injected on an analytical C18 column. The mobile phase was constituted by solvent A (0.5 % TFA in water) and solvent B (0.5 % TFA in 80 % acetonitrile in water). A linear gradient was used starting from 45% B, kept constant for 5 minutes, then the percentage of B was increased to 80% over 95 minutes, then raised to 100% B in 5 minutes. The HPLC profile showed a single peak at 57 min (64% B).The mass spectrometric analysis of the HPLC fraction containing the peak showed a single ion at 353, corresponding to $(M+H)^+$ of the MPS-I substrate.

Figure 10: General scheme for the synthesis of umbelliferone derivatives

Umbelliferone derivates were synthesized using a modified Pechmann condensation reaction of resorcinol with an ethyl-alkyl-ceto-acetate. The reaction is performed in concentrated sulfuric acid, at 100°C for 16 to 18 h. The example shows the condensation of a heavy ethyl acetoacetate with the formation of 4-methyl-umbelliferone-1,2,3,4-$^{13}$C used as internal standard for mass spectrometry, and a general reaction for the formation of 4-substitued umbelliferone derivatives.

Figure 11: Umbelliferone derivatives synthesized

List of umbelliferone derivatives synthesized for use in the synthesis of enzymatic substrates for quantification of LSD enzymes.

Figure 12: MS and MS/MS analysis of 4-ethyl-umbelliferone

MS analysis of $(M+H)^+$ ion at m/z 191 corresponding to 4-ethyl-umbelliferone. The isolation and fragmentation of this ion shows the formation of several fragments from which m/z 147 = $(M+H)^+$ -44 and 135 = $(M+H)^+$ -56 are the most important due to the similarity of the neutral loss with all umbelliferone derivatives.

Figure 13: MS and MS/MS analysis of 4-propyl-umbelliferone

MS analysis of the compound showing $(M+H)^+$ ion m/z 205 corresponding to 4-propyl-umbelliferone. The isolation and fragmentation of this ion shows the formation of several fragments from which 163 = $(M+H)^+$ -44 and 149 = $(M+H)^+$ - 56 are the most important due to the similarity of the neutral loss with all umbelliferone derivatives.

Figure 14: Umbelliferone derivatives fragmentation pattern elucidated by MS/MS analysis of 4-methyl-umbelliferone and 4-methyl-umbelliferone-1,2,3,4-$^{13}C$

MS/MS analysis of 4-methyl-umbelliferone and 4-methyl-umbelliferone-1,2,3,4-$^{13}C$ shows similar fragmentation spectra, shifted in the case of the heavy isotopic variants. The spectra show the similarity in neutral loss with other umbelliferone derivatives: -44, respectively -45 (loss of CO); -56, respectively -58 (loss of $C_2O$ moiety); -72, respectively -73 (loss of $C_3O_2$).

The present invention will now be further illustrated in the following examples without being limited thereto.

**Examples**

Experimental procedures:

*Preparation of dried blood spots (DBS).* Fresh blood or EDTA-treated blood is dropped (100 μL/spot) on a Whatman Protein Card. The blood drops are dried at 20°C for 24 h. DBS slides are cut with a puncher to slides of 3 mm diameter. The slides are stored at 20°C in 2.5 ml Eppendorf cups ready for use. A DBS slide with 3 mm diameter contains on average 1.5 μL plasma (with lysosomal activity), erythrocytes 1.5 μL (without lysosomal activity), 20,000 leucocytes (with lysosomal activity).

*Materials used for the fluorimetric and mass spectrometric determination of 4-alkyl umbelliferone.* DBS slides, diameter approx. 3 mm; substrate: 4-alkyl umbelliferyl derivatives; standard for fluorimetry: 4-alkyl umbelliferone; standard for mass spectrometry: 4-methyl umbelliferone 1,2,3,4-$^{13}C$ ; 5mM ammonium acetate buffer, pH 4; formic acid, analytical grade; 1 M glycine buffer, pH 9; Shaker incubator at 37°C; Black 96well plates and Eppendorf cups; Victor II Fluorimeter (Perkin Elmer); Bruker Esquire Ion Trap mass spectrometer with ESI-source.

Example 1 (Reference Example

Fluorimetric determination of lysosomal enzymatic activity

The reaction product of any lysosomal enzyme determined is a 4-alkyl umbelliferone (4-RU) which can be analyzed fluorimetrically at an excitation wavelength of 360 nm and emits at a wavelength of 400 nm. The product can be determined on any fluorimeter equipped with a specific umbelliferone filter. For quantification, a series of 12 dilutions of 4-RU with concentrations from 0 to 2.2 μM is used. A linear regression is performed for obtaining a calibration curve. All sample preparations are performed under red light due to the low photochemical stability of the 4-alkyl umbelliferyl derivatives at normal light.

The procedure for fluorimetric determination is carried out with the following steps (cf. Fig. 3):

1. 12 Dilutions of the standard are placed in an opaque 96 well plate at 100 μL/well.
2. DBSs are placed in the free wells in duplicates, wherein at least two healthy control samples are added to all patient samples.
3. Substrate solution (specific for each enzyme) is added to the DBSs at 100 μL/well. The substrate solutions are prepared in 0.5 mM ammonium acetate, pH 4, at a concentration of 1 μM.
4. The plate is sealed with a plate sealer to eliminate evaporation.
5. The plate is covered with aluminum foil to protect from light.
6. The plate is shaken for 45 min at 20°C.
7. The plate is centrifuged.
8. The seal is removed.
9. The plate is analyzed at 360 nm excitation and scanned at 400 nm. The results of the scan are considered blanks to be subtracted from the results of final scan.
10. The plate is sealed with a plate sealer to eliminate evaporation.
11. The plate is covered with aluminum foil to protect from light.
12. The plate is incubated for 12 to 48 h at 37°C in an incubator (the incubation time is specific for each enzyme

determination).
13. Following incubation, the plate is centrifuged and measured directly by fluorescence as above, or is further prepared for mass spectrometric determination (cf. Example 2). For mass spectrometric determination, the DBS are transferred from the wells to another plate.

Linear regression is performed to obtain a standard curve. The scan at concentration 0 is considered the background which is subtracted from the results at other concentrations. From the final samples, the start scan results are subtracted and the activity of the enzyme is obtained by extrapolating the differences against the standard curve. An enzymatic activity lower than 20% of the average enzymatic activity of the healthy control samples is considered as a deficient enzyme.

Example 2:

Mass spectrometric determination of lysosomal enzymatic activity

The reaction product of all reactions is 4-alkyl-umbelliferone with molecular masses varying from 162 to 218. As internal standard, 4-methyl-umbelliferone-1,2,3,4-$^{13}C$, (M.W. 181) is used. All umbelliferone derivates have specific MS/MS spectra, from which the following specific fragmentations are common to all:

1) 28 amu (neutral loss of -CO);
2) 44 amu (45 in case of the $^{13}C$-derivative, corresponding to neutral loss of $CO_2$);
3) 98 amu (99 in case of the $^{13}C$-derivative, corresponding to neutral loss of - $C_4H_2O_3$).

Parameters for MS determinations are specific for different types of mass spectrometers (e.g. ion trap or triple-quadrupole mass spectrometers), as well as for the type of measurement (e.g. single reaction monitoring mass spectrometry (SRM-MS) or multiple reaction monitoring mass spectrometry (MRM-MS). For triple-quadrupole instruments, the measurement of parent-daughter ions transitions is used for the product ion (or products in the case of multiplex assays) and for the internal standard. For ion trap instruments, the isolation and monitoring of the product parent ions and of the internal standard is used only to confirm the measured ions of the product and internal standard. For all mass spectrometric assays, no additional sample cleaning steps are required (such as desalting, liquid-liquid and solid-liquid extraction). The incubation solution is mixed with internal standard solution and directly injected into the electrospray (ESI) source of the mass spectrometer.
The following steps are used for mass spectrometric assay (cf. Fig. 4):

1. Steps 2, 3, 4, 5, 6, 7, 12, and 13 are identical to the fluorimetric assay (cf. Example 1).
2. 100 μL 10% formic acid solution (stop solution) is added to the DBS-incubation solution.
3. 100 μL standard solution (1 μM 4-methyl umbelliferone 1,2,3,4-$^{13}C$) is added.
4. The mixture containing 100 μL incubation solution + DBS, 100 μL stop solution and 100 μL standard solution is vortexed and then centrifuged at 130000 rtp.
5. The supernatant solution is injected into the mass spectrometer.

Examples of diagnostics by fluorimetric and mass spectrometric determination are shown in Figure 5 for the enzymatic activities of a healthy control sample and an MPS-I patient. Determinations of the enzymatic activities of a healthy control sample and an MPS-II patient by fluorimetry and ion trap mass spectrometry are shown in Figure 6. Determinations of enzymatic activities of a healthy control sample and a Fabry’s Disease patient by fluorimetry and by ion trap mass spectrometry’ are shown in Figure 7.

Example 3:

Experimental details for MPS-I diagnosis by fluorimetry and MRM-MS

The following experimental steps are performed:

1. DBSs prepared as described above from patients and healthy controls are introduced into Eppendorf cups or black ELISA plate wells in duplicates or triplicates.
2. 100 μL of 1 μM substrate in 5 mM ammonium acetate buffer is added in each cup/well on top of the DBS.
3. The cups/plate is/are covered with aluminum foil.
4. The cups/plate are agitated for 45 min at 20°C and then incubated for 6 to 48 h at 37°C.

5. The reaction is quenched by adding 10 μL formic acid (pH is kept below 2), preferably under red light.
6. Determination by fluorimetry is performed using an extrapolation with a standard curve using 4-methyl umbelliferone as external standard.
7. 90 μL of a fresh solution of 0.667 μM umbelliferone in 5 mM ammonium acetate buffer is added (final concentration of the internal standard for mass spectrometry is 0.3 μM).
8. The mixture is vortexed and centrifuged.
9. The supernatant is determined by MRM-MS, by monitoring either the MS ions of the product and internal standard (m/z 177 and 163), or the MS/MS ions (m/z 105 and 119).
10. The concentrations are calculated using the formula:

$$\frac{\text{Conc.}_{\text{Product}}}{\text{Intens.}_{\text{Product}}} = \frac{\text{Conc.}_{\text{Internal Std}}}{\text{Intens.}_{\text{Internal std}}}.$$

Example 4:

Synthesis of 4-methylumbelliferyl α-L-iduronide

A person with Hurler syndrome, also known as Mucopolysaccharidosis type I (MPS-I), does not have a viable α-L-iduronidase, the enzyme responsible for degradation of heparan sulfate and dermatan sulfate. Clinical diagnosis is performed by monitoring the formation of 4-methylumbelliferone in the presence of blood components from 4-methylumbelliferyl-α-L-iduronide. 4-Methylumbelliferyl-α-L-iduronide has the chemical formula $C_{16}H_{16}O_9$, desiccates at -20°C and is light-sensitive.

The synthesis starting from L-glucose is carried out in six steps to the final product, 4-methylumbelliferyl-α-L-iduronide (Fig. 8). In step 1, L-glucose is converted to penta-O-acetyl-L-glucose (Product 1). For this reaction, 5 g (0.027 mol) L-glucose, 2.5 g sodium acetate and 20 ml acetic anhydride were introduced into a 250 ml flask. The reaction mixture was heated to 45°C under agitation on a magnetic stirrer until homogenization was reached. After 30 minutes, 20 ml water was added and the flask cooled on ice. The solution was then filtrated through a Bruckner funnel and crystals obtained by adding 15 ml ice-cold 25% acetic acid solution. The crystals were dissolved in water, followed by drying *in vacuo* on a rotary evaporator. After removal of solvent, product **1** was obtained with a hazel, gel-like consistence. The reaction monitoring was performed by thin layer chromatography (TLC) using the mobile phase methanol:water (60:40). The visualization and characterization of the product was performed by ESI-MS at the following conditions: Positive ion mode, capillary-4500V, nebulizer, 15 psi, drying gas, 6 psi, drying temperature 200°C, skimmer 40 V; capillary end, 80 V. The mass spectrum showed the major ion, $(M+Na)^+$. After purification by liquid-liquid extraction, 7.5 g (0.019 mol) of product **1** was obtained. In step 2, penta-O-acetyl-L-glucose **1** was converted to 2,3,4,6,-tetra-O-acetyl-β-L-glucopyranoside-chloride **2.** 7.5 g **1** was dissolved in 600 ml dichloromethane (DCM) at 20°C under agitation; after 15 min, 0.5 g $AlCl_3$ was added, and a further 0.5 g $AlCl_3$ after another 15 min; after further 15 min 20 mL benzene and 0.3 g silicic acid were added, yielding a brown glue-like substance. The mixture was stirred for 30 min and the reaction product visualized by TLC (mobile phase, 50:50 DCM:hexane), and dried by vaporization at 70°C. The resulting brown powder was redissolved in DCM and 10% sodium sulfate, and product **2** isolated by liquid-liquid extraction from the DCM fraction. The DCM fraction was dried, and the raw product redissolved in 20 mL methanol:DCM (50:50). Purification was performed on a silica gel column, and fractions analyzed by TLC. The purified product (5 g; 0.013 mol) was obtained as a brown clay.

*Synthesis of 1,2,3,4,6, penta-O-acetyl-α-L-idopyranose (product **3**).*

Product **2** (ca. 5 g) was dissolved in 100 mL DCM at -10°C under an argon atmosphere. After 15 min, 25 ml $SbCl_5$ was added while maintaining a temperature of -10°C, which resulted in a green solution. After 30 min, the cooling bath was removed while the argon atmosphere was maintained. The precipitate was then dissolved in hot water, and DCM and ether were added to form a black precipitate. The resulting product was redissolved in DCM and 100 g sodium-potassium tartrate. Product **3** was isolated by liquid-liquid extraction from the middle fraction, yielding a white precipitate. The raw product was dried, redissolved in methanol and filtrated through a Bruckner funnel, yielding a white solid. It was again redissolved in DCM:methanol (1:1), and purification performed by silica gel chromatography. **3** was amorphous and did not show fluorescence; yield 3.1 g (0.007 mol).

In step 4, 1,2,3,4,6-penta-O-acetyl-α-L-idopyranose was condensed with 4-methylumbelliferone to obtain 4-MU-2,3,4,6-tetra-O-acetyl-α-L-idopyranoside **(4).** 3.1 g of 3 was mixed with 4 g 4-methylumbelliferone (MU) in 15 ml ethanol diacetate, and the reaction mixture heated to 135°C under agitation with a magnetic stirrer. After 30 min, 15 g ZnCb was added in 4 ml acetic acid:acetic anhydride (4:1) and the mixture was stirred for 2 hours. The temperature was then increased to 300°C for 30 min, yielding a brown foam. After 20 min, the mixture was treated with 100 ml 10% sodium

sulfate and DCM in an ice-bath. Product **4** was isolated by liquid-liquid extraction from the DCM fraction. The DCM fraction was dried, and the crude product redissolved in 30 ml methanol:DCM (1:1) and purified on a silica gel column; purity was ascertained by TLC (mobile phase, DCM:hexane, 1:1). The product was then further purified by preparative HPLC on a C18 column; final yield was 1.1 g (0.0021 mol) of **4** (white powder). In step 5, 4-MU-2,3,4,6-tetra-O-acetyl-α-L-idopyranoside was de-acetylated to obtain product **5** (4-methylumbelliferone-α-L-idopyranoside). 4-MU-2,3,4,6-tetra-O-acetyl-α-L-idopyranoside was dissolved in 20 ml DCM and heated to 200°C under agitation in an oil bath. After 30 min, 30 ml methanol was added, and after further 15 min, 0.12 ml 3 M sodium methoxide was added. After another 15 min the mixture was allowed to cool, neutralized with 30 ml acetic acid and was finally lyophilized.

4-Methylumbelliferyl-α-L-iduronide **6** was obtained by oxidizing **5.** 0.6 g MU-α-L-idopyranoside was dissolved in water, and 0.7 g Adams' catalyst {$PtO_2$) was added under compressed air, to maintain pH 5 by adding 30 ml sodium bicarbonate (3 mM) in water. After 60 min, another 0.5 g $PtO_2$ was added, yielding a grey solution. After further 60 min, the reaction was quenched and the product dried *in vacuo.* The product formation was controlled by TLC. The crude product was dissolved in 80% acetonitrile, 20% water (70 ml); the supernatant was lyophilized yielding a brown solid which was redissolved and purified by HPLC. The preparative HPLC was repeated 3 times to obtain the final pure product; 5.2 mg (0.0000147 mol). The homogeneity of the MPS-I substrate was analyzed by ESI-ion trap mass spectrometry, and provided a single ion at m/z 353, corresponding to $(M+H)^+$ (Fig. 9).

<u>Example 5:</u>

<u>Synthesis of umbelliferone derivatives</u>

Umbelliferone derivatives were generally synthesized by a modified Pechmann condensation with conc. $H_2SO_4$ which was required as a catalyst for the condensation of resorcinol with an ethyl-alkylacetate (Fig. 10, 11). As examples, the synthesis of 4-methyl-umbelliferyl-1,2,3,4-$^{13}C$, 4-ethyl-umbelliferone, 4-propyl-umbeliferone and 4-*t*-butyl-umbelliferone are described in the following.

*4-Methyl-umbelliferyl-1,2,3,4-$^{13}C$.*

500 mg (3.84 mmol) of ethyl-aceto-acetate-1,2,3,4-$^{13}C$ and 875 mg (7.95 mmol) resorcinol were placed in a 200 ml glass reactor, and 25 ml sulfuric acid added and placed on a thermo-magnetic stirrer for 16 h at 90°C. The solution was then cooled in an ice bath and 25 ml ice cold water was added slowly until a slurry precipitate formed. To the mixture 50 ml ether was dropped and the precipitate redissolved. The ether fraction was dried *in vacuo.* For re-crystallization, water was added and the crystals collected by filtration in a Buchner funnel. The precipitate was washed yielding a white final product; purity was confirmed by ESI-ion trap mass spectrometry, showing a single ion at m/z 181 corresponding to $(M+H)^+$. The yield of the product was 70% (480mg).

*4-Ethyl-umbelliferone.*

5 g (0.035 mol) ethyl-propionyl-acetate and 7.7 g (0.070 mol) resorcinol were mixed as described above, and 50 mL sulfuric acid added and reacted for 18 h on a thermo-magnetic stirred at 90°C. The solution was cooled on ice and 50 ml ice cold water added until a slurry precipitate formed. 100 ml ether was then added to dissolve the precipitate. The ether fraction was dried, and water added for re-crystallization; crystals were collected by filtration on a Buchner funnel. The precipitate was washed until a final white-yellowish product was obtained; purity was confirmed by ESI-ion trap MS, providing an ion signal at m/z 191 corresponding to $(M+H)^+$ (Fig. 12). Yield: 68%.

*4-Propyl-umbelliferone.*

5 g (0.1031 mol) ethyl-propionyl-acetate and 6.93 g (0.063 mol) resorcinol were reacted with 50 ml sulfuric acid as described above (18 h at 90°C). The solution was cooled down on ice and product precipitated with 50 ml ice cold water. After addition of 100 ml diethylether, the ether fraction was dried. For re-crystallization, water was added and the crystals collected by filtration. The final product was white-yellow and purity was confirmed by ESI-ion trap MS (Fig. 13); final yield, 75%.

*4-t-Butyl-umbelliferone.*

5 ml (0.029 mol) ethyl-propionyl-acetate and 6.39 g (0.058 mol) resorcinol were reacted with 50 ml sulfuric acid for 17 h at 90°C. The solution was cooled on ice and the precipitate redissolved with 100 ml ether. For re-crystallization, water was added and the crystals were collected by filtration. The final product was a brown, viscous liquid and purity

confirmed by ESI-ion trap MS (m/z 219; $(M+H)^+$; yield 55%.

Fragmentation patterns of umbelliferone derivatives elucidated by MS/MS analysis are shown for 4-methyl-umbelliferone and 4-methyl-umbelliferone-1,2,3,4-$^{13}C$ in Fig. 14.

## Claims

1. An in vitro method for the diagnosis of a lysosomal storage disease (LSD) in a subject, comprising the steps of:

    (a) providing a blood sample of the subject;
    (b) incubating the blood sample in the presence of more than one 4-alkyl umbelliferyl derivatives according to formula (I):

    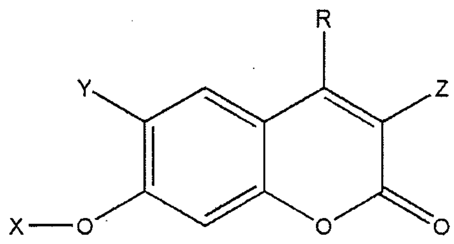

    (I)

    wherein:

        R is selected from the group consisting of H, methyl, ethyl, propyl, isopropyl, tert.-butyl, pentyl, hexyl, and octyl;
        X is sulfate or a sugar moiety that is specifically recognized by a lysosomal enzyme, deficiency of which is the cause of an LSD; and
        Y and Z are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15; and

    wherein said 4-alkyl umbelliferyl derivatives all differ from each other in both substituents R and X, but are identical in both substituents Y and Z;
    (c) determining the amount of more than one 4-alkyl umbelliferones corresponding in the substituent R, Y, and Z to the 4-alkyl umbelliferyl derivative used in step (b) in the sample by mass spectrometry; and
    (d) assessing the activity of the more than one lysosomal enzymes specifically recognizing the sugar moiety or sulfate X based on the amounts of 4-alkyl umbelliferone determined in step (c).

2. The method of claim 1, wherein the LSD is selected from the group consisting of Type II Glycogen Storage Diseases, Mucopolysaccharidoses (MPSs), Mucolipidoses (MLs), Oligosaccharidoses, Lipidoses, and Sphingolipidoses.

3. The method of claim 2, wherein the LSD is an MPS, selected from the group consisting of Hurler syndrome (Mucopolysaccharidosis type I; MPS-I), Hunter syndrome (MPS-II), Sanfilippo syndrome A (MPS-IIIA), Sanfilippo syndrome B (MPS-IIIB), Sanfilippo syndrome C (MPS-IIIC), Sanfilippo syndrome D (MPS-IIID), classic Morquio syndrome (MPS-IVA), Morquio B Disease (MPS-IVB), Maroteaux-Lamy syndrome (MPS-VI), and Sly syndrome (MPS-VII).

4. The method of claim 2, wherein the LSD is an ML, selected from the group consisting of Sialidosis (Mucolipidosis type I; ML-I), I-cell Disease (ML-II), and Pseudo-Hurler Polydystrophy (ML-III).

5. The method of claim 2, wherein the LSD is a Sphingolipidosis, selected from the group consisting of Fabry's Disease, Niemann-Pick-Disease Type A, Niemann-Pick Disease Type B, Gaucher's Disease, Krabbe's Disease, GM1 Gangliosidosis, Tay-Sachs Disease, Sandhoff Disease, Metachromatic Leukodystrophy, Farber Disease, Multiple sulfatase deficiency, and Galactosialidosis.

6. The method of any one of claims 1 to 5, wherein the LSD and the corresponding substituent X in formula (I) are at least one of Pompe disease and α-glucose; MPS-I and α-1-iduronide; MPS-II and α-iduronate-2-sulfate; MPS-IIIA and 2-sulfamino-2-deoxy-α-D-glucopyranoside; MPS-IIIB and 2-acetamido-2-deoxy-α-D-glucopyranoside; MPS-IIIC and α-D-2-amino-glucopyranoside; MPS-IIID and 2-acetamido-2-deoxy-6-sulfate-β-D-glucopyranoside; MPS-IVA and β-D-galactopyranoside-6-sulfate sodium salt; MPS-IVB and β-D-galactopyranoside; MPS-VI and sulfate; MPS-VII and β-D-glucuronide; ML-I and α-D-N-acetyl neuraminic acid; ML-II and one of β-D-glucuronide, N-acetylglucosamine, β-D-galactoside, and α-L-fucoside; ML-III and α-D-manno-pyranoside; Schindler Disease/Kanzaki Disease and α-N-acetylgalactosamine; α-Mannosidosis and α-D-mannose; β-Mannosidosis and β-mannose; α-Fucosidosis and α-L-fucose; Aspartylglucosaminuria and aspartylglucosamine; Niemann-Pick Disease Type C and cholesterol; Niemann-Pick Disease Type D and shingolipids; Neuronal ceroid lipofuscinoses and the amino-terminal tripeptidyl derivative GFFLAF; Wolman Disease and short chain fatty-acyl esters such as palmitate, eloidate, lignocerate and cholesteryl esters; Fabry's Disease and α-D-galactopyranoside; Gaucher's Disease and β-D-glucopyranoside; Niemann-Pick Disease Type A and phosphocholine; Niemann-Pick Disease Type B and phosphocholine; Krabbe's Disease and β-D-galactopyranoside; GM1-Gangliosidosis and β-D-galactopyranoside; Tay-Sachs Disease and α-N-acetylglucosamine; Sandhoff Disease and β-N-acetylglucosamine; Metachromatic Leukodystrophy and sulfate; Farber Disease and ceramide; Multiple sulfatase deficiency and sulfate; and Galactosialidosis and N-acetylneuraminic acid or β-galactose.

**7.** The method of any one of claims 1 to 6, wherein the blood sample is selected from the group consisting of whole blood, serum, plasma, and dried blood spots (DBS).

**8.** The method of any one of claims 1 to 7, wherein the incubation in step (b) is for 12 to 48 hours at 37°C.

**9.** The method of any one of claims 1 to 8, wherein the detection in step (c) comprises both fluorimetric detection and detection by mass spectrometry.

**10.** The method of any one of claims 1 to 9, wherein an LSD is diagnosed when the activity of the respective lysosomal enzyme causing said LSD is at least 20% lower than the average activity of said enzyme in healthy control samples.

**11.** A 4-alkyl umbelliferyl derivative according to formula (I):

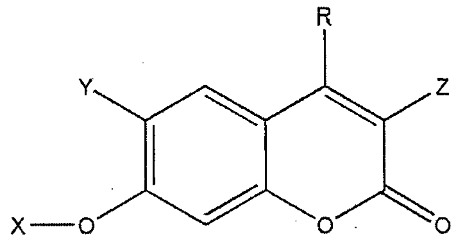

(I)

wherein:

R is selected from the group consisting of H, methyl, ethyl, propyl, isopropyl, tert.-butyl, pentyl, hexyl, and octyl;
X is sulfate or a sugar moiety that is specifically recognized by a lysosomal enzyme, deficiency of which is the cause of an LSD; and
Y and Z are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15;

for use in the diagnosis of an LSD.

**12.** Use of a 4-alkyl umbelliferyl derivative according to formula (I):

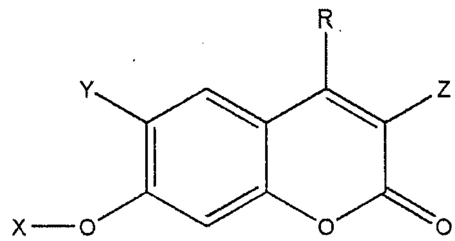

(I)

wherein:

R is selected from the group consisting of H, methyl, ethyl, propyl, isopropyl, tert.-butyl, pentyl, hexyl, and octyl;
X is sulfate or a sugar moiety that is specifically recognized by a lysosomal enzyme, deficiency of which is the cause of an LSD; and
Y and Z are independently H, $-OCH_3$, $-CH_3$, $-C_2H_5$, $-C_3H_7$, $-OC_2H_5$, or $-NH-CO-(CH_2)_n-CH_3$, wherein n is 1, 3, 5, 7, 9, 11, 13 or 15;

in the in vitro diagnosis of an LSD.

**13.** A kit for the diagnosis of a lysosomal storage disease (LSD) in a subject, comprising at least one of the 4-alkyl umbelliferyl derivatives as defined in claim 11.

**Patentansprüche**

**1.** *In vitro* Verfahren für die Diagnose einer lysosomalen Speicherkrankheit *(lysosomal storage disease;* LSD) in einem Subjekt, umfassend die Schritte:

(a) Bereitstellen einer Blutprobe des Subjekts,
(b) Inkubieren der Blutprobe in Anwesenheit mehr als eines 4-Alkylumbelliferylderivats nach Formel (I):

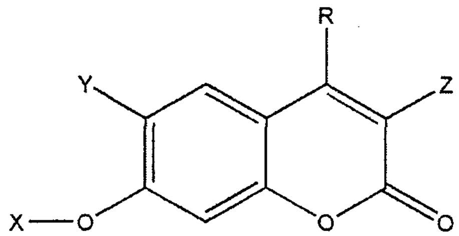

(I)

wobei:

R ausgewählt ist aus der Gruppe, bestehend aus H, Methyl, Ethyl, Propyl, Isopropyl, tert.-Butyl, Pentyl, Hexyl und Octyl,
X Sulfat ist oder eine Zuckereinheit, die spezifisch durch ein lysosomales Enzym erkannt wird, dessen Defizienz die Ursache einer LSD ist und
Y und Z unabhängig voneinander H, $-OCH_3$, $-CH_3$, $-C_2H_5$, $-C_3H_7$, $-OC_2H_5$ oder $-NH-CO-(CH_2)_n-CH_3$ sind, wobei n 1, 3, 5, 7, 9, 11, 13 oder 15 ist und wobei die 4-Alkylumbelliferylderivate sich alle in beiden Substituenten R und X voneinander unterscheiden, aber in beiden Substituenten Y und Z identisch sind,

(c) Bestimmen der Menge von mehr als einem 4-Alkylumbelliferon in der Probe durch Massenspektrometrie, das in den Substituenten R, Y und Z dem in Schritt (b) verwendeten 4-Alkylumbelliferylderivat entspricht und
(d) Beurteilen der Aktivität des mehr als einem lysosomalen Enzyms, das spezifisch die Zuckereinheit oder das Sulfat X erkennt, basierend auf den in Schritt (c) bestimmten Mengen von 4-Alkylumbelliferon.

**2.** Verfahren nach Anspruch 1, wobei die LSD ausgewählt ist aus der Gruppe, bestehend aus Glycogenspeichererkrankungen Typ II, Mucopolysaccharidosen (MPS), Mukolipidosen (ML), Oligosaccharidosen, Lipidosen und Sphingolipidosen.

**3.** Verfahren nach Anspruch 2, wobei die LSD eine MPS ist, ausgewählt aus der Gruppe, bestehend aus Hurler-Syndrom (Mucopolysaccharidose Typ I, MPS-I), Hunter-Syndrom (MPS-II), Sanfilippo-Syndrom A (MPS-IIIA), Sanfilippo-Syndrom B (MPS-IIIB), Sanfilippo-Syndrom C (MPS-IIIC), Sanfilippo-Syndrom D (MPS-IIID), klassischem Morquio-Syndrom (MPS-IVA), Morbus Morquio Typ B (MPS-IVB), Maroteaux-Lamy-Syndrom (MPS-VI) und Sly-Syndrom (MPS-VII).

**4.** Verfahren nach Anspruch 2, wobei die LSD eine ML ist, ausgewählt aus der Gruppe, bestehend aus Sialidose (Mucolipidose Typ I, ML-I), I-Zellkrankheit (ML-II) und Pseudo-Hurler-Polydystrophie (ML-III).

**5.** Verfahren nach Anspruch 2, wobei die LSD eine Sphingolipidose ist, ausgewählt aus der Gruppe, bestehend aus

Morbus Fabry, Morbus Niemann-Pick Typ A, Morbus Niemann-Pick Typ B, Morbus Gaucher, Morbus Krabbe, Gangliosidose, Morbus Tay-Sachs, Morbus Sandhoff, metachromatischer Leukodystrophie, Morbus Farber, multipler Sulfatasedefizienz und Galactosialidose.

**6.** Verfahren nach einem der Ansprüche 1 bis 5, wobei die LSD und der entsprechende Substituent X in Formel (I) zumindest eines aus Morbus Pompe und α-Glucose, MPS-I und α-1-Iduronid, MPS-II und α-Iduronat-2-Sulfat, MPS-IIIA und 2-Sulfamino-2-deoxy-α-D-glucopyranosid, MPS-IIIB und 2-Acetamido-2-deoxy-α-D-glucopyranosid, MPS-IIIC und α-D-2-amino-glucopyranosid, MPS-IIID und 2-Acetamido-2-deoxy-6-sulfat-β-D-glucopyranosid, MPS-IVA und β-D-Galactopyranosid-6-sulfatnatriumsalz, MPS-IVB und β-D-Galactopyranosid, MPS-VI und Sulfat, MPS-VII und β-D-Glucoronid, ML-I und α-D-N-Acetylneuraminsäure, ML-II und eines aus β-D-Glucoronid, N-Acetylglucosamin, β-D-Galactosid und α-L-Fucosid, ML-III und α-D-Mannopyranosid, Morbus Schindler/Morbus Kanzaki und α-N-Acetylgalactosamin, α-Mannosidose und α-D-Mannose, β-Mannosidose und β-Mannose, α-Fucosidose und α-L-Fucose, Aspartylglucoseaminurie und Aspartylglucosamin, Morbus Niemann-Pick Typ C und Cholesterin, Morbus Niemann-Pick Typ D und Shingolipide, neuronale ceroide Lipofuscinosen und das aminoterminale Tripeptidylderivat GFFLAF, Morbus Wolman und kurzkettige Fettsäureacylester wie Palmitat, Eloidat, Lignocerat und Cholesterylester, Morbus Fabry und α-D-Galactopyranosid, Morbus Gaucher und β-D-Glucopyranosid, Morbus Niemann-Pick-Typ A und Phosphocholin, Morbus Niemann-Pick-Typ B und Phosphocholin, Morbus Krabbe und β-D-Galactopyranosid, GM1-Gangliosidose und β-D-Galactopyranosid, Morbus-Tay-Sachs und α-N-Acetylglucosamin, Morbus Sandhoff und β-N-Acetylglucosamin, metachromatische Leukosystrophie und Sulfat, Morbus Farber und Ceramid, multiple Sulfatasedefizienz und Sulfat und Galactosialidose und N-Acetylneuraminsäure oder β-Galactose ist.

**7.** Verfahren nach einem der Ansprüche 1 bis 6, wobei die Blutprobe ausgewählt ist aus der Gruppe, bestehend aus Vollblut, Serum, Plasma und getrockneten Blutflecken *(dried blood spots,* DBS).

**8.** Verfahren nach einem der Ansprüche 1 bis 7, wobei das Inkubieren in Schritt (b) für 12 bis 48 Stunden bei 37°C erfolgt.

**9.** Verfahren nach einem der Ansprüche 1 bis 8, wobei der Nachweis in Schritt (c) sowohl fluorimetrischen Nachweis als auch Nachweis durch Massenspektrometrie umfaßt.

**10.** Verfahren nach einem der Ansprüche 1 bis 9, wobei eine LSD diagnostiziert wird; wenn die Aktivität des betreffenden lysosomalen Enzyms, das die LSD verursacht, mindestens 20% niedriger als die durchschnittliche Aktivität des Enzyms in gesunden Kontrollproben ist.

**11.** 4-Alkylumbelliferylderivat nach Formel (I):

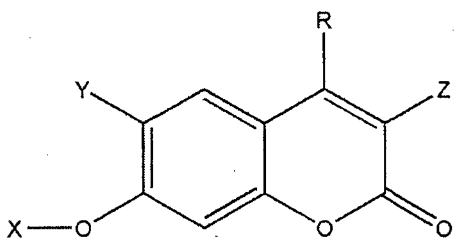

(I)

wobei:

R ausgewählt ist aus der Gruppe, bestehend aus H, Methyl, Ethyl, Propyl, Isopropyl, tert.-Butyl, Pentyl, Hexyl und Octyl,
X Sulfat ist oder eine Zuckereinheit, die spezifisch durch ein lysosomales Enzym erkannt wird, dessen Defizienz die Ursache einer LSD ist und
Y und Z unabhängig voneinander H, $-OCH_3$, $-CH_3$, $-C_2H_5$, $-C_3H_7$, $-OC_2H_5$ oder $-NH-CO-(CH_2)_n-CH_3$ sind, wobei n 1, 3, 5, 7, 9, 11, 13 oder 15 ist,

zur Verwendung in der Diagnose einer LSD.

**12.** Verwendung eines 4-Alkylumbelliferylderivats nach Formel (I):

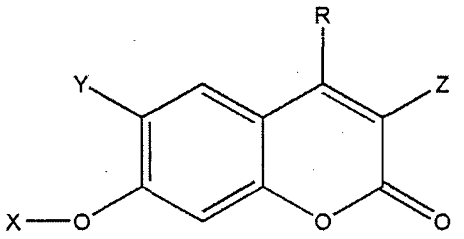

(I)

wobei:

R ausgewählt ist aus der Gruppe, bestehend aus H, Methyl, Ethyl, Propyl, Isopropyl, Tert.-Butyl, Pentyl, Hexyl und Octyl,
X Sulfat ist oder eine Zuckereinheit, die spezifisch durch ein lysosomales Enzym erkannt wird, dessen Defizienz die Ursache einer LSD ist und
Y und Z unabhängig voneinander H, $-OCH_3$, $-CH_3$, $-C_2H_5$, $-C_3H_7$, $-OC_2H_5$ oder $-NH-CO-(CH_2)_n-CH_3$ sind, wobei n 1, 3, 5, 7, 9, 11, 13 oder 15 ist,

in der *in vitro* Diagnose einer LSD.

**13.** Kit für die Diagnose einer lysosomalen Speicherkrankheit (*lysosomal storage disease;* LSD) in einem Subjekt, umfassend mindestens eines der 4-Alkylumbelliferylderivate wie in Anspruch 11 definiert.

## Revendications

**1.** Procédé in vitro pour le diagnostic d'une maladie de stockage lysosomal (MSL) chez un sujet, comprenant les étapes de :

(a) fourniture d'un échantillon de sang du sujet ;
(b) incubation de l'échantillon de sang en présence de plus d'un dérivé de 4-alkyl-umbelliféryle selon la formule (I)

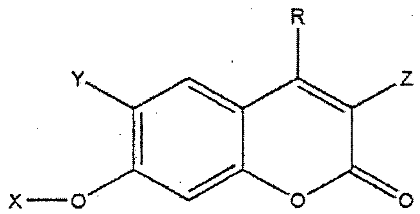

(I)

dans laquelle :

R est choisi dans le groupe constitué de H, méthyle, éthyle, propyle, isopropyle, tert-butyle, pentyle, hexyle et octyle ;
X est un sulfate ou un groupement de sucre qui est spécifiquement reconnu par une enzyme lysosomales, dont la déficience est la cause d'une MSL ; et
Y et Z sont indépendamment H, $-OCH_3$, $-CH_3$, $-C_2H_5$, $-C_3H_7$, $-OC_2H_5$ ou $-NH-CO-(CH_2)_n-CH_3$, où n vaut 1, 3, 5, 7, 9, 11, 13 ou 15 ; et
dans lequel lesdits dérivés de 4-alkyl-umbelliféryle diffèrent tous les uns des autres au niveau des deux substituants R et X, mais sont identiques au niveau des deux substituants Y et Z ;

(c) détermination de la quantité du plus d'une 4-alkyl-umbelliférone correspondant au niveau des substituants R, Y et Z au dérivé de 4-alkyl-umbelliféryle utilisé dans l'étape (b) dans un échantillon par spectrométrie de masse ; et
(d) évaluation de l'activité de la plus d'une enzyme lysosomale reconnaissant spécifiquement le groupement de sucre ou le sulfate X d'après les quantités de 4-alkyl-umbelliférone déterminées dans l'étape (c).

2. Procédé selon la revendication 1, dans lequel la MSL est choisie dans le groupe constitué des maladies de stockage du glycogène de type II, des mucopolysaccharidoses (MPS), des mucolipidoses (ML), des oligosaccharidoses, des lipidoses et des sphingolipidoses.

3. Procédé selon la revendication 2, dans lequel la MSL est une MPS, choisie dans le groupe constitué du syndrome de Huler (mucopolysaccharidose de type I ; MPS-1), du syndrome de Hunter (MPS-II), du syndrome de Sanfilippo A (MPS-IIIA), du syndrome de Sanfilippo B (MPS-IIIB), du syndrome de Sanfilippo C (MPS-IIIC), du syndrome de Sanfilippo D (MPS-IIID), du syndrome de Morquio classique (MPS-IVA), de la maladie de Morquio B (MPS-IVB), du syndrome de Moreteaux-Lamy (MPS-VI) et du syndrome de Sly (MPS-VII).

4. Procédé selon la revendication 2, dans lequel la MSL est une ML, choisie dans le groupe constitué de la sialodose (mucolipidose de type I ; ML-I), de la maladie des cellules I (ML-II) et de la polydystrophie de Pseudo-Hurler (ML-III).

5. Procédé selon la revendication 2, dans lequel la MSL est une sphingolipidose, choisie dans le groupe constitué de la maladie de Fabry, de la maladie de Niemann-Pick de type A, de la maladie de Niemann-Pick de type B, de la maladie de Gaucher, de la maladie de Krabbe, de la gangliosidose GM1, de la maladie de Tay-Sachs, de la maladie de Sandhoff, de la leucodystrophie métachromatique, de la maladie de Farber, de la déficience multiple en sulfatase et de la galactosialidose.

6. Procédé selon l'une quelconque des revendications 1 à 5, dans lequel la MSL et le substituant X correspondant dans la formule (I) sont l'un au moins parmi la maladie de Pompe et l'α-glucose ; la MPS-I et l'α-1-iduronide ; la MPS-II et l'α-iduronate-2-sulfate ; la MPS-IIIA et le 2-sulfamino-2-désoxy-α-D-glucopyranoside ; la MPS-IIIB et le 2-acétamino-2-désoxy-α-D-glucopyranoside ; la MPS-IIIC et l'α-D-2-alino-glucopyranoside ; la MPS-IIID et le 2-acétamido-2-désoxy-6-sulfate-β-D-glucopyranoside ; la MPS-IVA et le sel de sodium du β-D-galactopyranoside-6-sulfate ; la MPS-IVB et le β-D-galactopyranoside ; la MPS-VI et le sulfate ; la MPS-VII et le β-D-glucoronide ; la ML-1 et l'acide α-D-N-acétyl-neuraminique ; la ML-II et l'un parmi le β-D-glucoronide, la N-acétyl-glucosamine, le β-D-galactoside et l'α-L-fucoside ; la ML-III et l'α-D-Manno-pyranoside ; la maladie de Schindler/maladie de Kanzaki et l'α-N-acétylgalactosamine ; l'α-mannosidose et l'α-D-mannose ; la β-mannosidose et la β-mannose ; l'α-fucosidose et l'α-Lf-ucose ; l'aspartyl-glucosaminurie et l'aspartylglucosamine ; la maladie de Niemann-Pick de type C et le cholestérol ; la maladie de Niemann-Pick de type D et les shingolimides ; les lipofuscinoses céroïdes neuronales et le dérivé de tripeptidyle amino-terminal GFFLAF ; la maladie de Wolman et les acyl-esters gras à chaîne courte tel que le palmitate, l'éloidate, le lignocérate et les cholestérylesters ; la maladie de Fabry et l'a-D-galactopyranoside ; la maladie de Gaucher et le β-D-glucopyranoside ; la maladie de Niemann-Pick de type A et la phosphocholine ; la maladie de Niemann-Pick de type B et la phosphocholine ; la maladie de Krabbe et le β-D-glucopyranoside ; la gangliosidose GM1 et la β-D-glucopyranoside ; la maladie de Tay-Sachs et l'α-N-acétylglucosamine ; la maladie de Sandhoff et la β-N-acétylglucosamine ; la leucodystrophie métachromatique et le sulfate ; la maladie de Farber et le céramide ; la déficience multiple en sulfatase et le sulfate ; et la galactosialidose et l'acide N-acétylneuraminique ou le β-galactose.

7. Procédé selon l'une quelconque des revendications 1 à 6, dans lequel l'échantillon sanguin est choisi dans le groupe constitué du sang complet, du sérum, du plasma et de taches de sang séchées (TSS).

8. Procédé selon l'une quelconque des revendications 1 à 7, dans lequel l'incubation dans l'étape (b) dure de 12 à 48 heures à 37°C.

9. Procédé selon l'une quelconque des revendications 1 à 8, dans lequel la détection dans l'étape (c) comprend à la fois une détection fluorimétrique et une détection par spectrométrie de masse.

10. Procédé selon l'une quelconque des revendications 1 à 9, dans lequel la MSL est diagnostiquée lorsque l'activité de l'enzyme lysosomale respective provoquant ladite MSL est inférieure d'au moins 20 % à l'activité moyenne de ladite enzyme dans des échantillons témoins sains.

11. Dérivé de 4-alkyl-umbelliféryle selon la formule (I) :

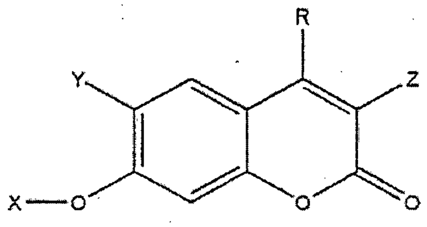

(I)

dans laquelle :

R est choisi dans le groupe constitué de H, méthyle, éthyle, propyle, isopropyle, tert-butyle, pentyle, hexyle et octyle ;
X est un sulfate ou un groupement de sucre qui est spécifiquement reconnu par une enzyme lysosomale, dont la déficience est la cause d'une MSL ; et
Y et Z sont indépendamment H, $-OCH_3$, $-CH_3$, $-C_2H_5$, $-C_3H_7$, $-OC_2H_5$ ou $-NH-CO-(CH_2)_n-CH_3$, où n vaut 1, 3, 5, 7, 9, 11, 13 ou 15 ;

pour une utilisation dans le diagnostic d'une LSD.

**12.** Utilisation d'un dérivé de 4-alkyl-umbelliféryle selon la formule (I) :

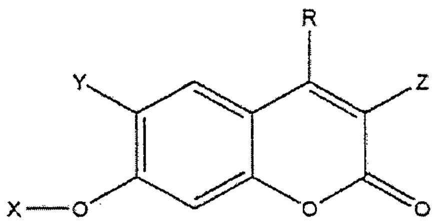

(I)

dans laquelle :

R est choisi dans le groupe constitué de H, méthyle, éthyle, propyle, isopropyle, tert-butyle, pentyle, hexyle et octyle ;
X est un sulfate ou un groupement de sucre qui est spécifiquement reconnu par une enzyme lysosomale, dont la déficience est la cause d'une MSL ; et
Y et Z sont indépendamment H, $-OCH_3$, $-CH_3$, $-C_2H_5$, $-C_3H_7$, $-OC_2H_5$ ou $-NH-CO-(CH_2)_n-CH_3$, où n vaut 1, 3, 5, 7, 9, 11, 13 ou 15 ;

dans le diagnostic d'une LSD.

**13.** Kit de diagnostic d'une maladie de stockage lysosomal (MSL) chez un sujet, comprenant l'un au moins des dérivés de 4-alkyl-umbelliféryle définis dans la revendication 11.

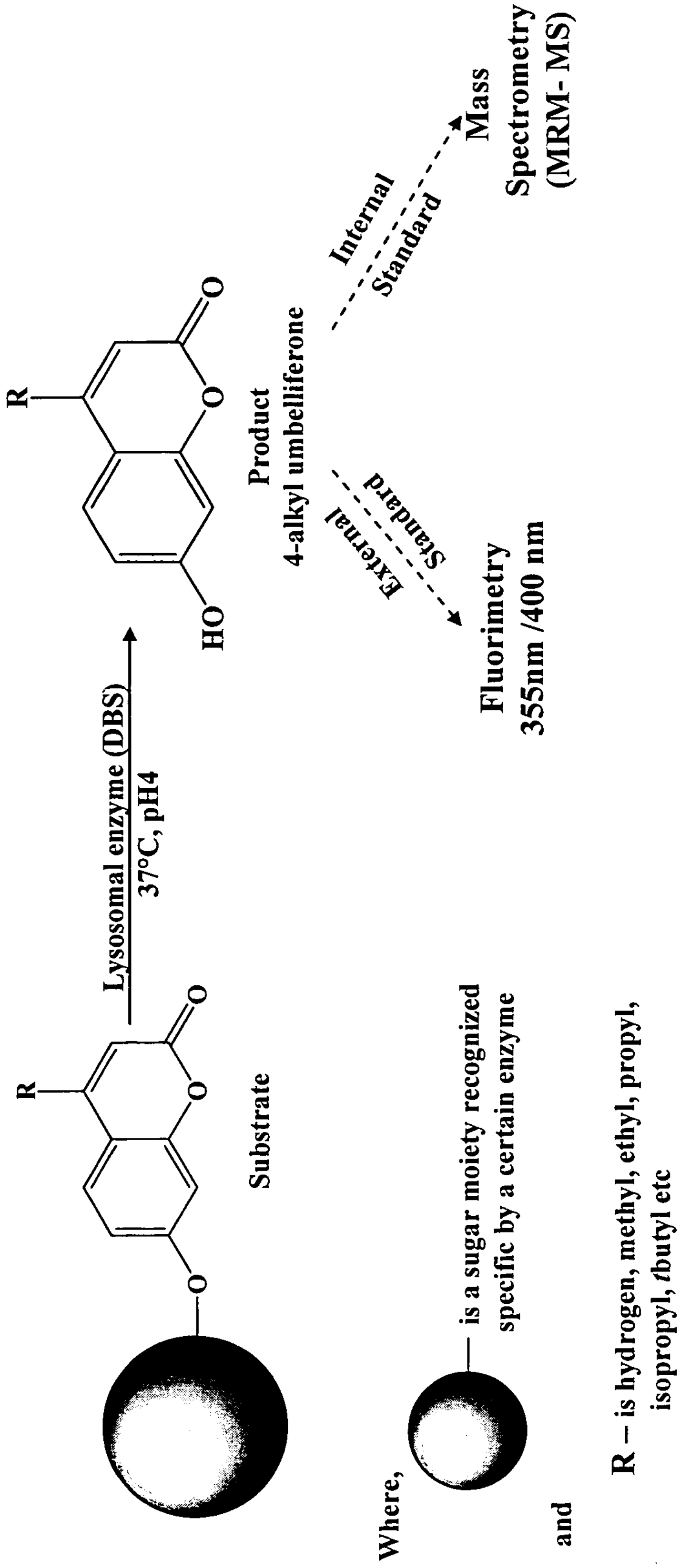
Substrate
Lysosomal enzyme (DBS)
37°C, pH4
Product
4-alkyl umbelliferone
External Standard
Internal Standard
Fluorimetry
355nm /400 nm
Mass Spectrometry
(MRM- MS)
Where,
is a sugar moiety recognized specific by a certain enzyme
and
R – is hydrogen, methyl, ethyl, propyl, isopropyl, tbutyl etc

Figure 1

| *Disease* | Determined enzyme | Substrate analog (name) | Substrate analog (Chemical Formula) |
|---|---|---|---|
| *3-1. MPSI Hurler syndrome* | α-L-iduronidase | 4-alkyl umbelliferyl α-l-iduronide MPS I S | 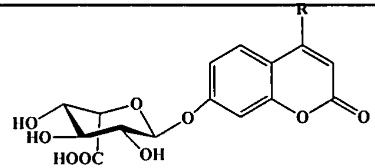 |
| *3-2. MPS II Hunter syndrome* | Iduronate sulfatase | 4-alkyl-umbelliferyl-α-iduronate 2-sulphate MPS II S | 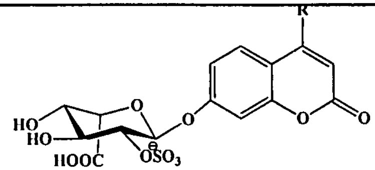 |
| *3-3. MPS III Sanfilippo syndrome A* | Heparan sulfamidase | 4-alkyl umbelliferyl 2-Sulfamino-2-deoxy-α-D-glucopyranoside MPS IIIA S | 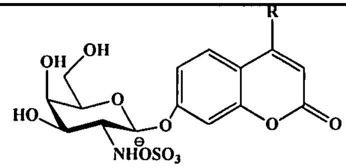 |
| *3-4. MPS III Sanfilippo syndrome B* | N-acetylglucosaminidase | 4-alkyl umbelliferyl-2-acetamido-2-deoxy-α-D-glucopyranoside. MPS IIIB S | 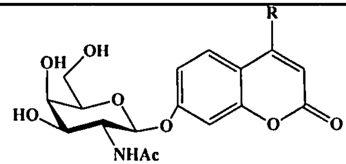 |
| *3-5. MPS III Sanfilippo syndrome C* | Acetyl-CoA:alpha-glucosaminide acetyltransferase | 4-alkyl umbelliferyl-α-D-2amino glucopyranoside MPS IIIC S | 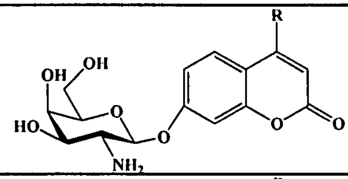 |
| *3-6. MPS III Sanfilippo syndrome D* | N-acetylglucosaminidas e 6-sulfatase | 4-alkyl umbelliferylL-2-acetamido-2-deoxy-6-sulphate-β-D-glucopyranoside MPS IIID S | 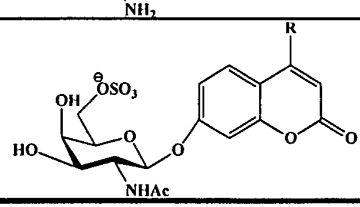 |

Figure 2

| *Disease* | Determined enzyme | Substrate analog (name) | Substrate analog (Chemical Formula) |
|---|---|---|---|
| *3-7. MPS IV Morquio syndrome A* | Galactose-6-sulfate sulfatase | 4-alkyl umbelliferyl Beta-D-galactopyranoside-6-sulfate sodium salt MPS IV a S | 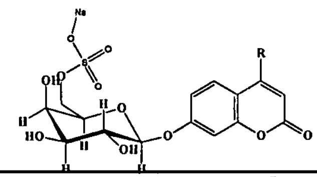 |
| *3-8. MPS IV Morquio syndrome B* | Beta-galactosidase | *4-alkyl umbelliferyl β-D-galactopyranoside* MPS IV b S | 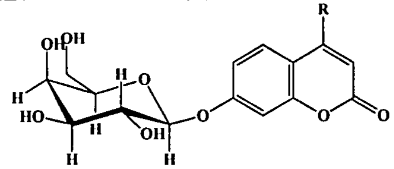 |
| *3-9. MPS VI Maroteaux-Lamy syndrome* | N-acetylgalactosamine-4-sulfatase | 4-alkyl umbelliferyl sulfate MPS VI S | 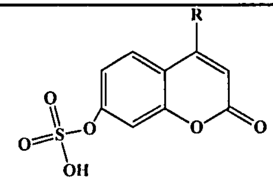 |
| *3-10. MPS VII Sly syndrome* | β-glucuronidase | 4-alkyl umbelliferyl-beta-D-glucuronide MPS VII S | 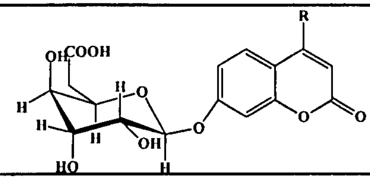 |
| *1. Fabry Disease* | α galactosidase | 4-alkyl umbelliferyl-α-D-galactopyranoside F S | 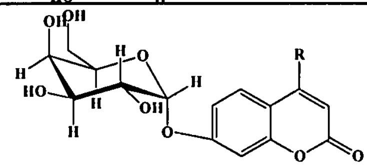 |
| *2. Gaucher Disease* | ß-glucocerebrosidase | 4-alkyl umbelliferyl-ß-D-glucopyranoside G S | 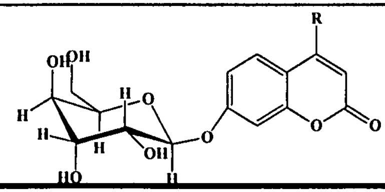 |

Figure 2 (cont.)

| *Disease* | *Determined enzyme* | *Substrate analog (name)* | *Substrate analog (formula)* |
|---|---|---|---|
| Mucolipidosis I (ML I) | Sialidase | 4-alkyl-umbelliferyl- *N*-Acetylneuraminic acid ML1S | 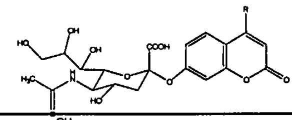 |
| Schindler Disease / Kanzaki Disease | N-acetylgalactosaminidase | 4-alkyl-umbelliferyl- α-N-acetylgalactosamine SKS | 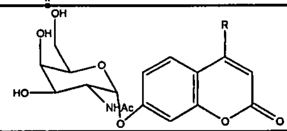 |
| α-Mannosidosis | α- D-mannosidase | 4-alkyl-umbelliferyl- α- D mannose | 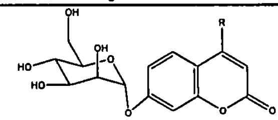 |
| β-Mannosidosis | β-mannosidase | 4-alkyl-umbelliferyl- β - mannose | 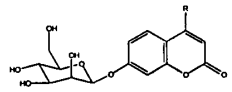 |
| α-Fucosidosis | α- L-fucosidase | 4-alkyl-umbelliferyl- α- L fucose | 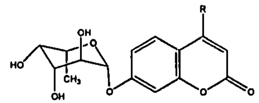 |
| Aspartylglucosaminuria | Aspartylglucosaminidase | 4-alkyl-umbelliferyl- Aspartylglucosamine | 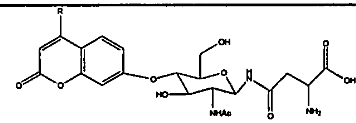 |
| Niemann-Pick A and B | Acid sphyngomyelinase | 4-methylumbelliferyl-phosphorylcholine | 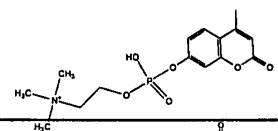 |
| Krabbe disease | β -galactosylceramidase | 6-hexadecanoylamino-4-umbelliferyl-beta-D galactopyranoside | 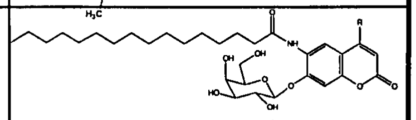 |
| GM1 gangliosidosis | β -galactosidase | 6-hexadecanoylamino-4-umbelliferyl-beta-D galactopyranoside | 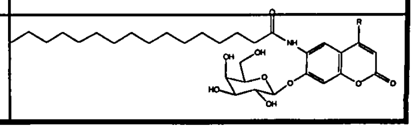 |

Figure 2 (cont.)

| *Disease* | *Determined enzyme* | *Substrate analog (name)* | *Substrate analog (formula)* |
|---|---|---|---|
| Tay Sachs Disease | Hexosaminidase A | 4-alkyl-umbelliferyl- β -N-acetylglucosamine | 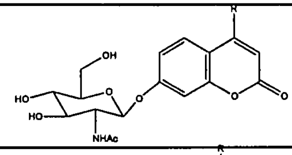 |
| Sandhoff disease | Hexosaminidase A and b | 4-alkyl-umbelliferyl- β-N-acetylglucosamine | 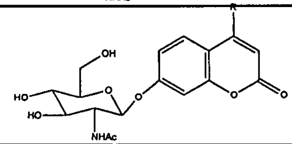 |
| Metachromatic Leukodystrophy | Arylsulfatese A | 4-alkyl-umbelliferyl- sulphate | 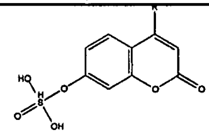 |
| Farber Disease | Ceramidase | 4-alkyl-umbelliferyl-ceramide | 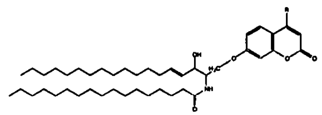 |
| multiple sulfatase deficiency | Sulphatase-modifying factor 1 | 4-alkyl-umbelliferyl- sulphate | 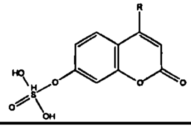 |
| Galactosialidosis | Cathepsin A<br>(tests for β-galactosidase and neuraminidase) | 4-alkyl-umbelliferyl- *N*-Acetylneuraminic acid and<br>4-alkyl-umbelliferyl- β- galactose | 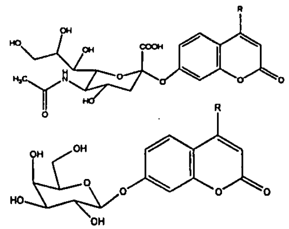 |
| Pompe disease | α- glucosidase | 4-alkyl-umbelliferyl- α- glucose | 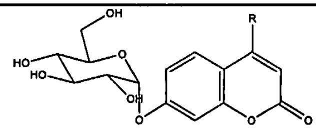 |

Figure 2 (cont.)

| *Disease* | *Determined enzyme* | *Substrate analog (name)* | *Substrate analog (formula)* |
|---|---|---|---|
| Acid lipase disease | Lipase | 4-methyl-umbelliferyl- nanoate | 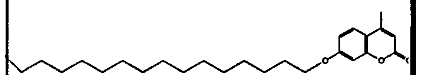 |
| ML 2+3 | Phosphotransferase | 4-methyl-umbelliferyl- phosphate | 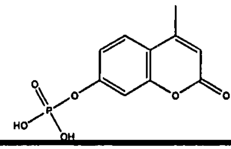 |

Figure 2 (cont.)

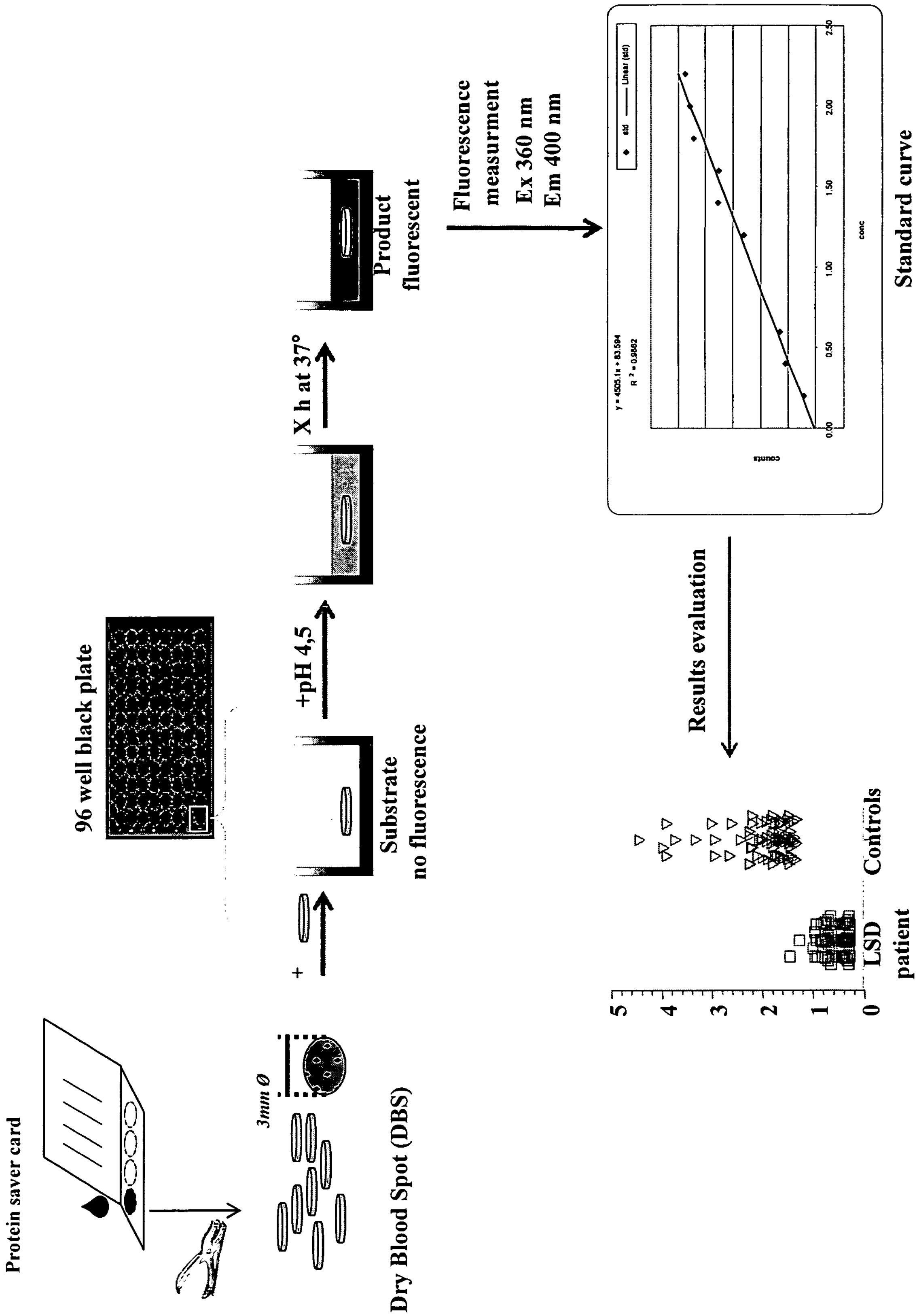
Protein saver card
3mm Ø
Dry Blood Spot (DBS)
96 well black plate
+
Substrate
no fluorescence
+pH 4,5
X h at 37°
Product
fluorescent
Fluorescence
measurment
Ex 360 nm
Em 400 nm
Standard curve
Results evaluation
LSD
patient
Controls

Figure 3

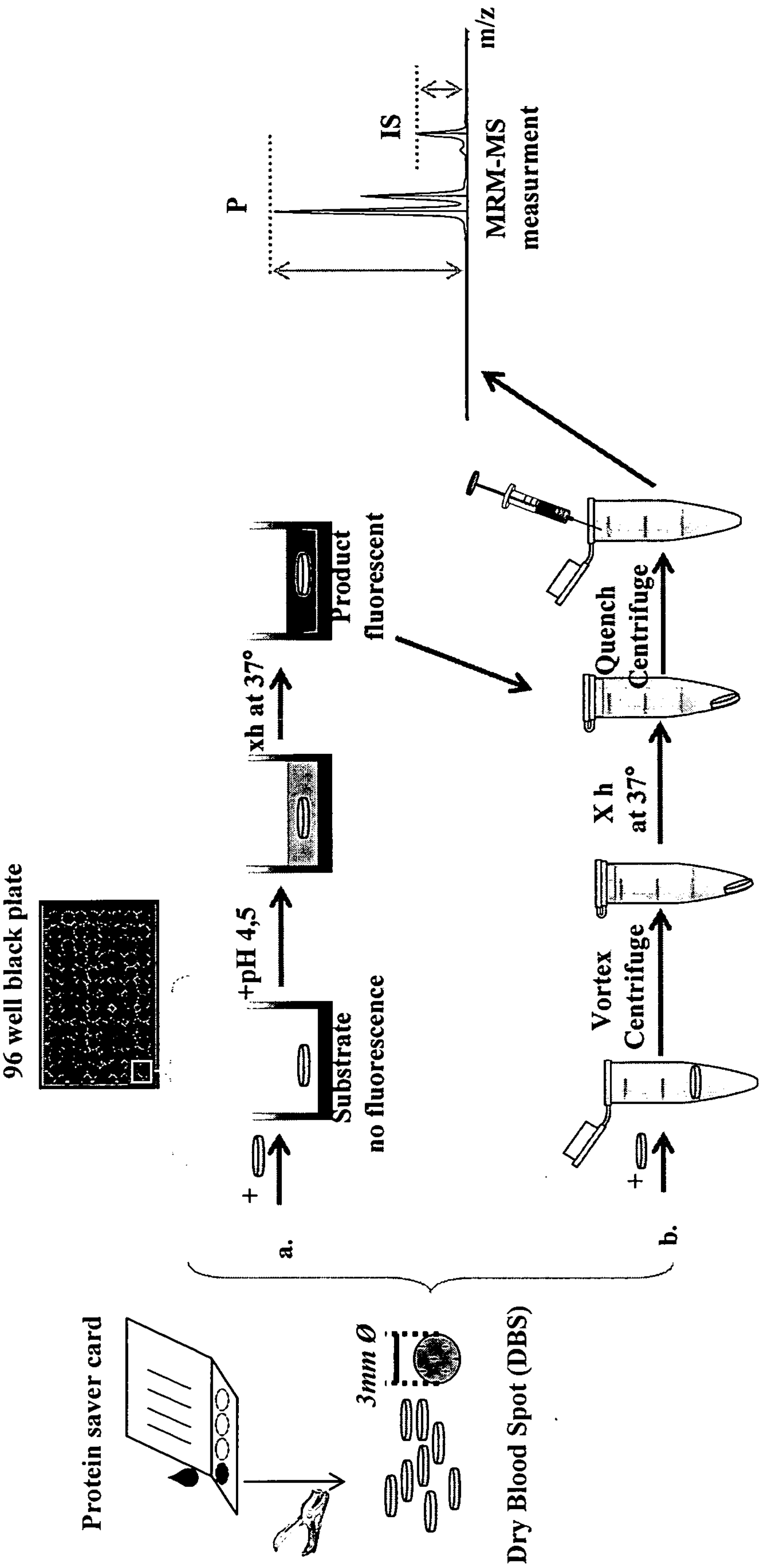
Protein saver card
Dry Blood Spot (DBS)
3mm Ø
a.
b.
96 well black plate
+
Substrate
no fluorescence
+pH 4,5
xh at 37°
Product
fluorescent
+
Vortex
Centrifuge
X h
at 37°
Quench
Centrifuge
P
IS
m/z
MRM-MS
measurment

Figure 4

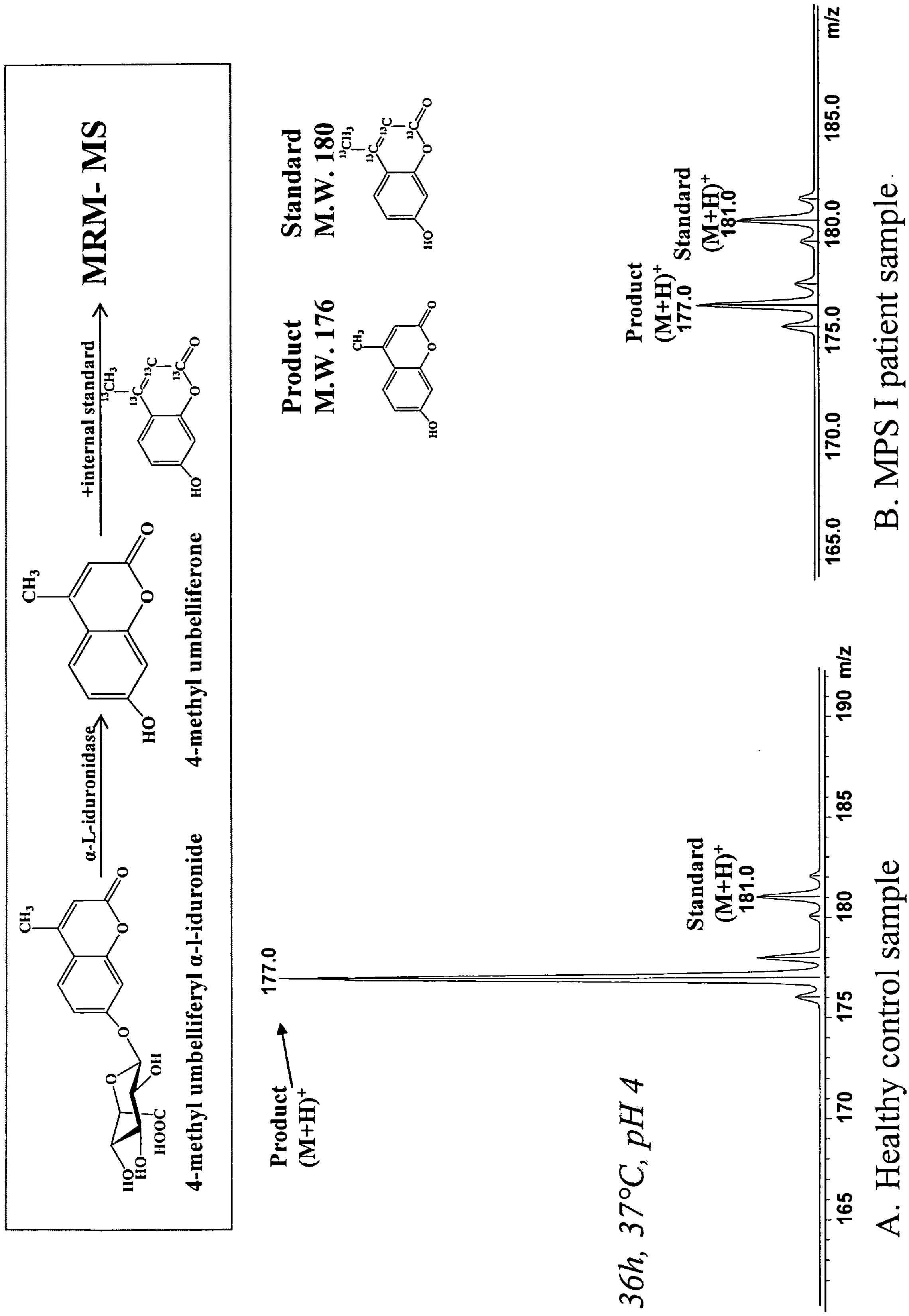
4-methyl umbelliferyl α-l-iduronide
α-L-iduronidase
4-methyl umbelliferone
+internal standard
MRM- MS
Product
M.W. 176
Standard
M.W. 180
36h, 37°C, pH 4
Product
(M+H)+
177.0
Standard
(M+H)+
181.0
165
170
175
180
185
190
m/z
A. Healthy control sample
Product
(M+H)+
177.0
Standard
(M+H)+
181.0
165.0
170.0
175.0
180.0
185.0
m/z
B. MPS I patient sample

Figure 5

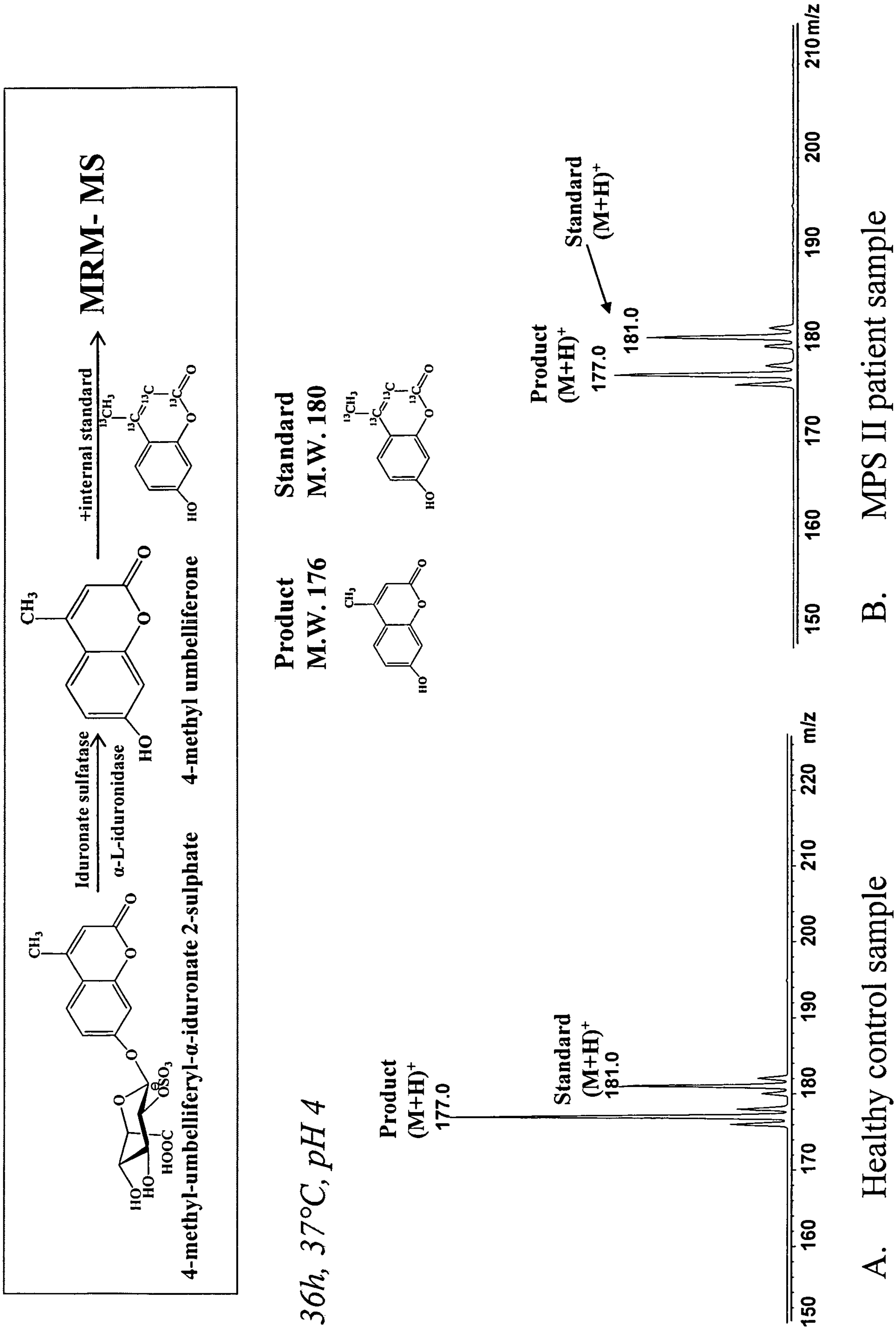
4-methyl-umbelliferyl-α-iduronate 2-sulphate
Iduronate sulfatase
α-L-iduronidase
4-methyl umbelliferone
+internal standard
MRM- MS
Product
M.W. 176
Standard
M.W. 180
36h, 37°C, pH 4
Product
(M+H)+
177.0
Standard
(M+H)+
181.0
A. Healthy control sample
B. MPS II patient sample
m/z

Figure 6

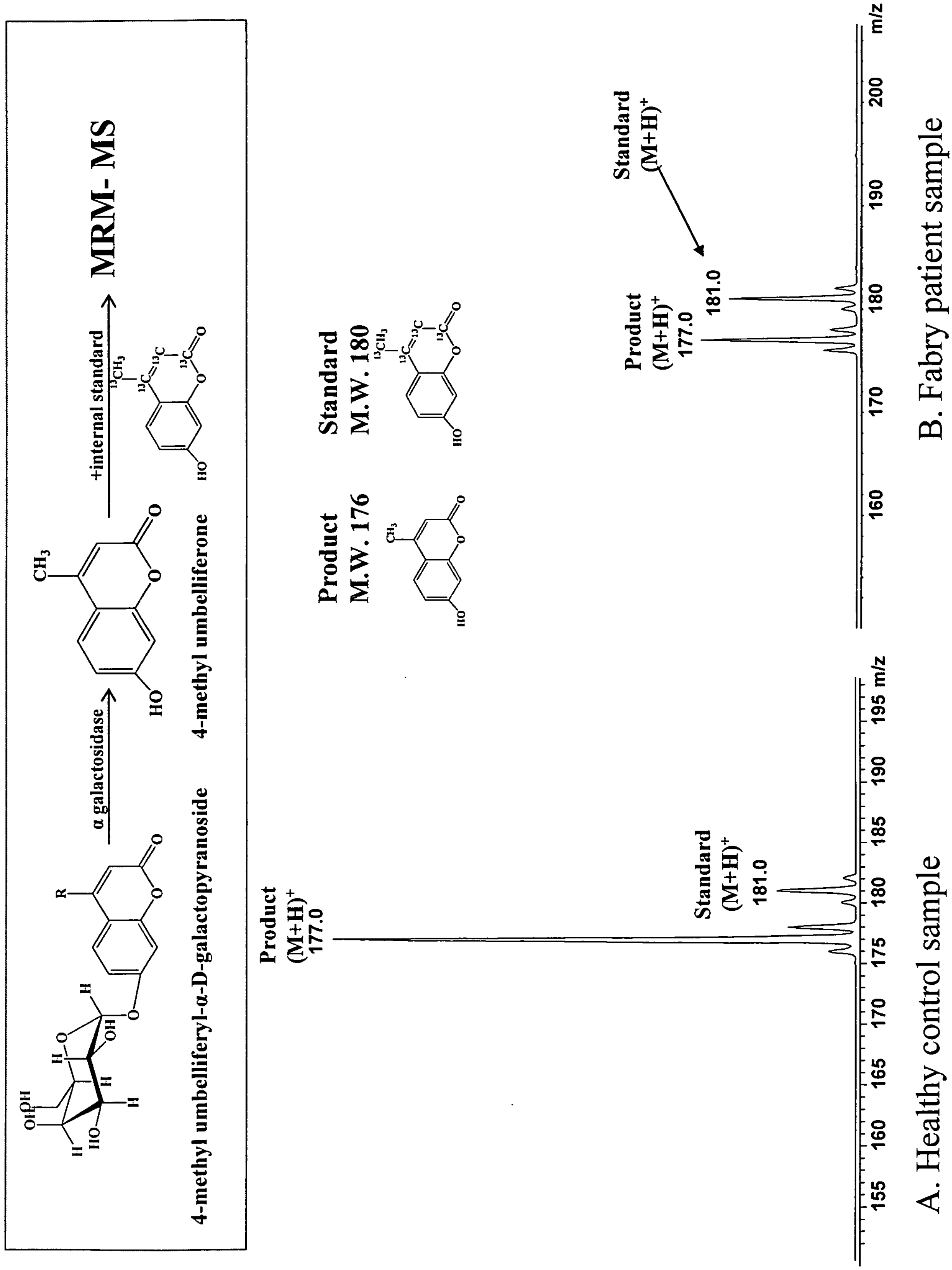
4-methyl umbelliferyl-α-D-galactopyranoside
α galactosidase
4-methyl umbelliferone
+internal standard
MRM- MS
Product
M.W. 176
Standard
M.W. 180
Product
$(M+H)^+$
177.0
Standard
$(M+H)^+$
181.0
155 160 165 170 175 180 185 190 195 m/z
A. Healthy control sample
Product
$(M+H)^+$
177.0
181.0
Standard
$(M+H)^+$
160 170 180 190 200 m/z
B. Fabry patient sample

Figure 7

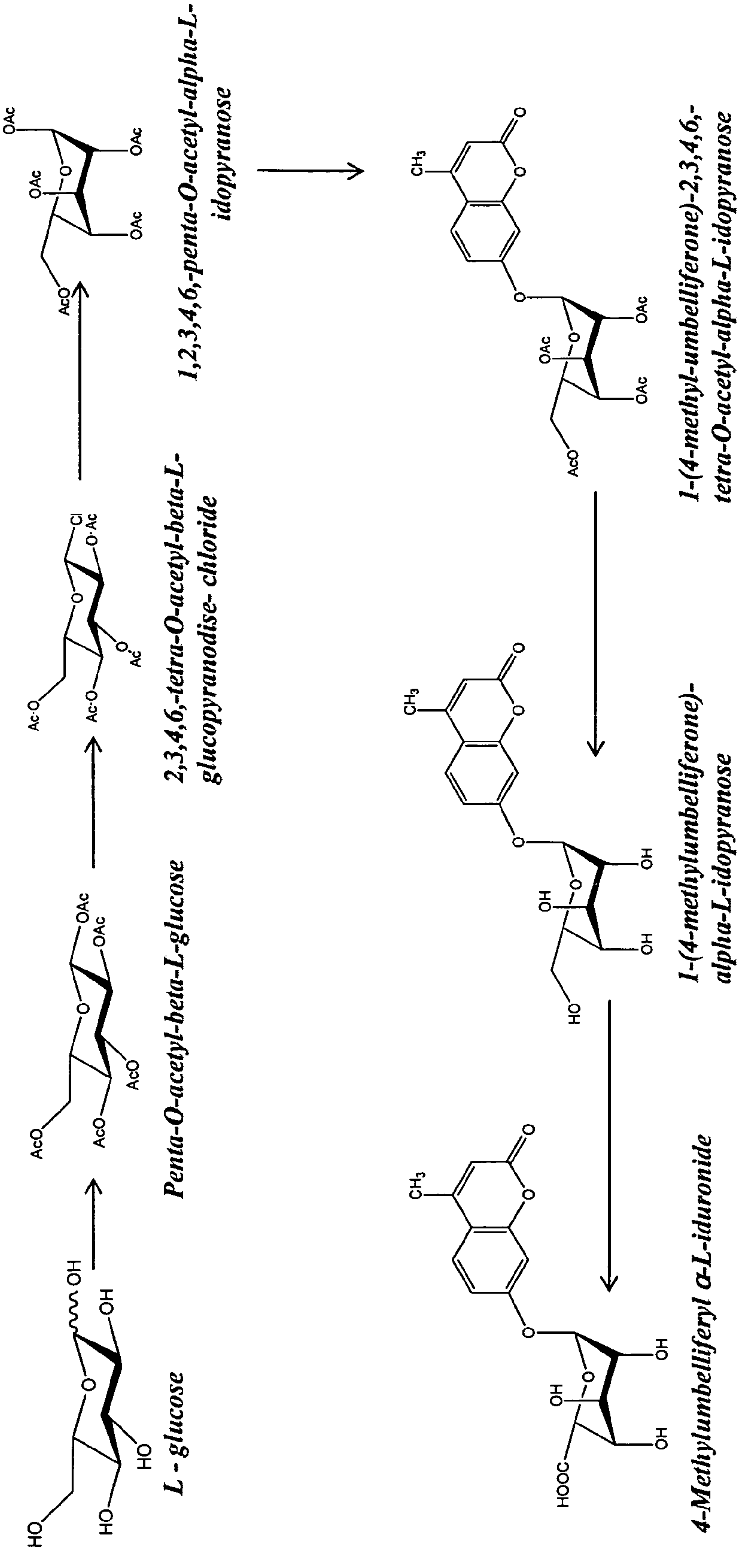

*L - glucose*

*Penta-O-acetyl-beta-L-glucose*

*2,3,4,6,-tetra-O-acetyl-beta-L-glucopyranodise- chloride*

*1,2,3,4,6,-penta-O-acetyl-alpha-L-idopyranose*

*1-(4-methyl-umbelliferone)-2,3,4,6,-tetra-O-acetyl-alpha-L-idopyranose*

*1-(4-methylumbelliferone)-alpha-L-idopyranose*

*4-Methylumbelliferyl α-L-iduronide*

Figure 8

*4-Methylumbelliferyl α-L-iduronide*

**MW. 352**

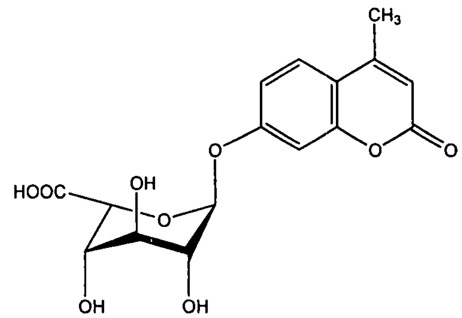

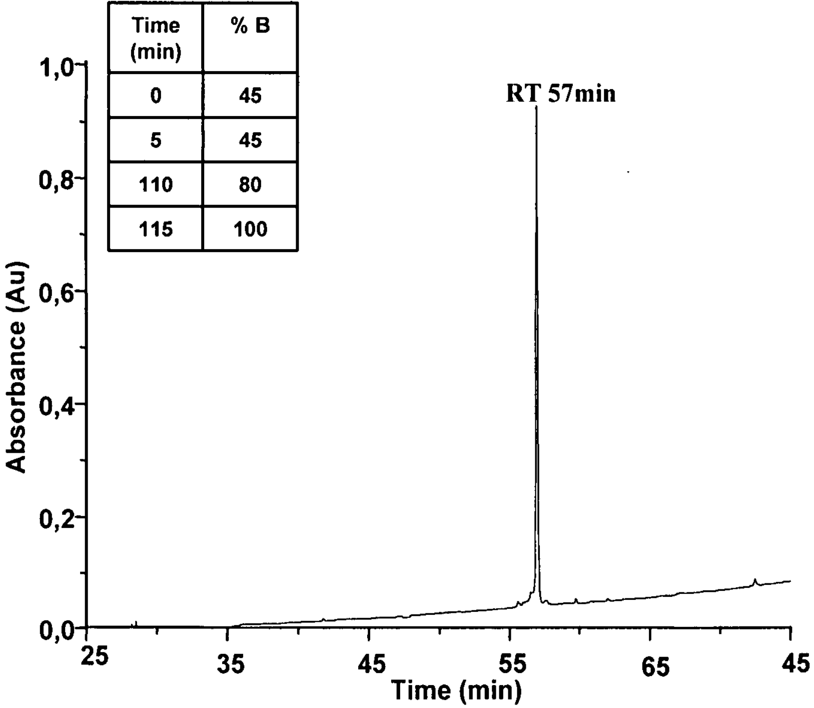


| Time (min) | % B |
|---|---|
| 0 | 45 |
| 5 | 45 |
| 110 | 80 |
| 115 | 100 |

**HPLC profile of 4-Methylumbelliferyl α-L-iduronide**

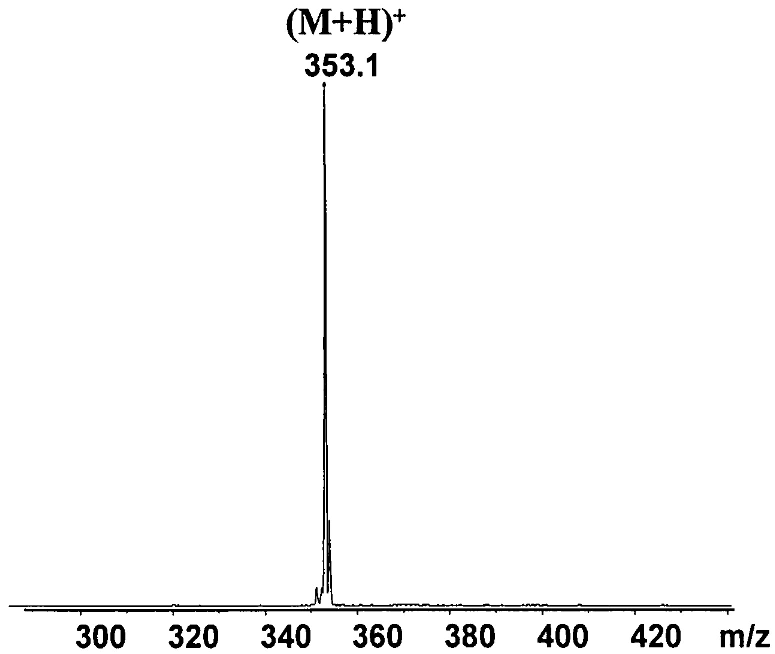


**Mass spectrum of 4-Methylumbelliferyl α-L-iduronide**

Figure 9

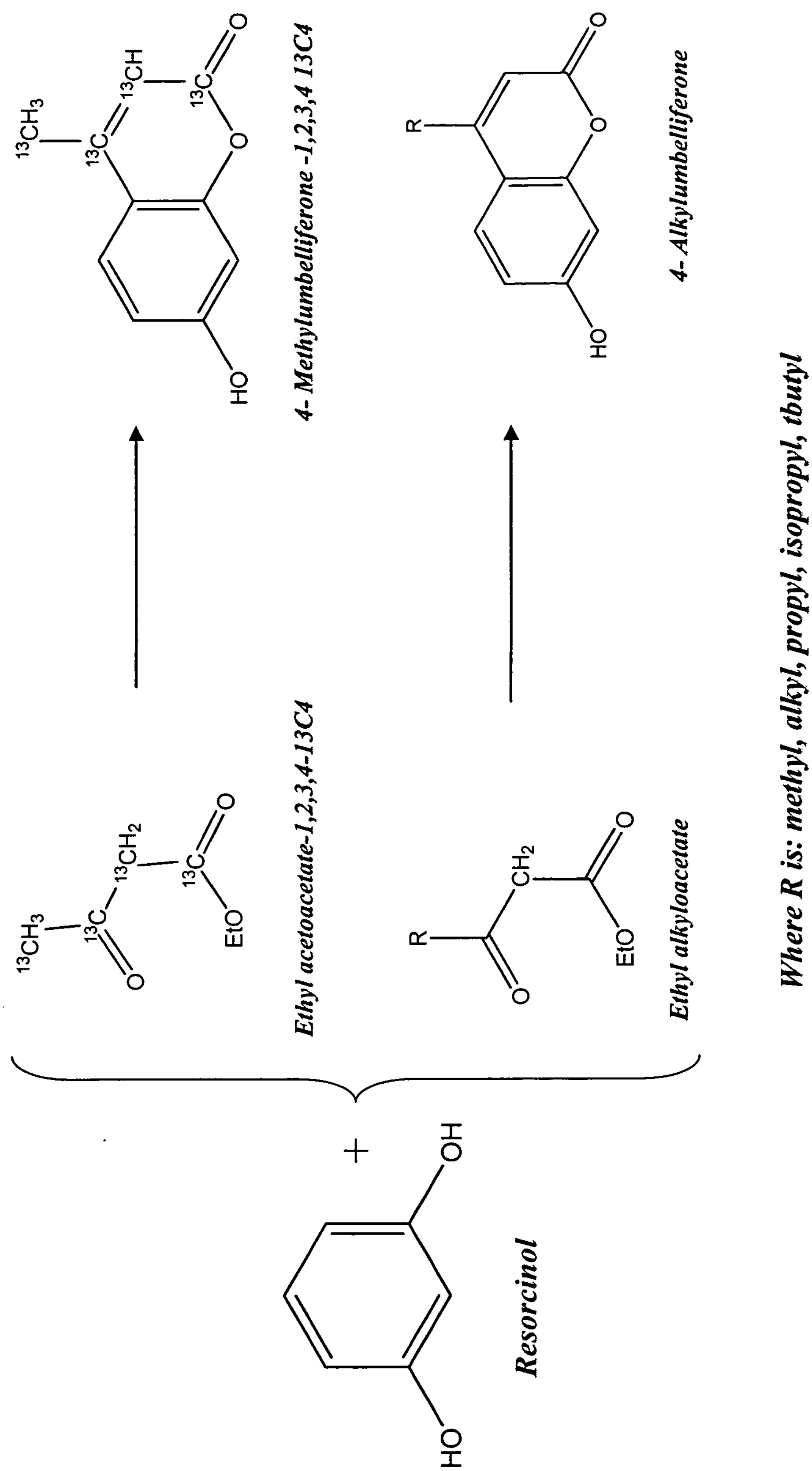

Resorcinol

Ethyl acetoacetate-1,2,3,4-13C4

Ethyl alkyloacetate

4- Methylumbelliferone -1,2,3,4 13C4

4- Alkylumbelliferone

Where R is: methyl, alkyl, propyl, isopropyl, tbutyl

Figure 10

| NR CRT | Umbelliferone derivates (name) | Formula (name) | Chemical analysis | Observation |
|---|---|---|---|---|
| 1 | Umbelliferone | 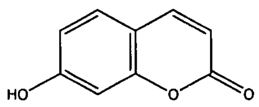 | Chemical Formula: $C_9H_6O_3$<br>Exact Mass: 162.03<br>Molecular Weight: 162.14<br>m/z: 162.03 (100.0%), 163.04 (9.9%), 164.04 (1.1%)<br>Elemental Analysis: C, 66.67; H, 3.73; O, 29.60 | White powder |
| 2 | 4- Methyl umbelliferone | 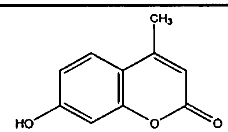 | Chemical Formula: $C_{10}H_8O_3$<br>Exact Mass: 176.05<br>Molecular Weight: 176.17<br>m/z: 176.05 (100.0%), 177.05 (11.0%), 178.05 (1.2%)<br>Elemental Analysis: C, 68.18; H, 4.58; O, 27.25 | White powder |
| 3 | 4- Methyl umbelliferone - 1,2,3,4 $^{13}$C4 | 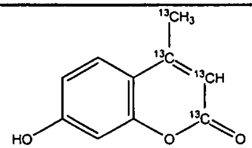 | Chemical Formula: $C_6{}^{13}C_4H_8O_3$<br>Exact Mass: 180.06<br>Molecular Weight: 180.14<br>m/z: 180.06 (100.0%), 181.06 (6.6%)<br>Elemental Analysis: C, 68.88; H, 4.48; O, 26.65 | White powder |
| 4 | 4-ethyl umbelliferone | 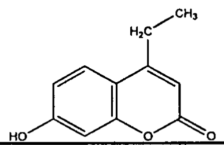 | Chemical Formula: $C_{11}H_{10}O_3$<br>Exact Mass: 190.06<br>Molecular Weight: 190.20<br>m/z: 190.06 (100.0%), 191.07 (12.1%), 192.07 (1.3%)<br>Elemental Analysis: C, 69.46; H, 5.30; O, 25.24 | Amorphous , dirty white |
| 5 | 4-propyl umbelliferone | 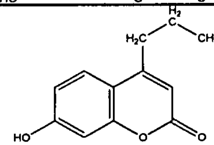 | Chemical Formula: $C_{12}H_{12}O_3$<br>Exact Mass: 204.08<br>Molecular Weight: 204.22<br>m/z: 204.08 (100.0%), 205.08 (13.2%)<br>Elemental Analysis: C, 70.57; H, 5.92; O, 23.50 | Amorphous , light yellow |
| 6 | 4-*iso*propyl umbelliferone | 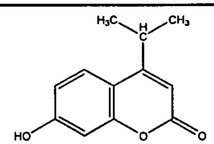 | Chemical Formula: $C_{12}H_{12}O_3$<br>Exact Mass: 204.08<br>Molecular Weight: 204.22<br>m/z: 204.08 (100.0%), 205.08 (13.2%)<br>Elemental Analysis: C, 70.57; H, 5.92; O, 23.50 | Bown liquid |
| 7 | 4-*tert*butyl umbelliferone | 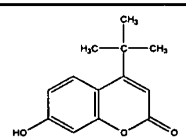 | Chemical Formula: $C_{13}H_{14}O_3$<br>Exact Mass: 218.09<br>Molecular Weight: 218.25<br>m/z: 218.09 (100.0%), 219.10 (14.3%), 220.10 (1.6%)<br>Elemental Analysis: C, 71.54; H, 6.47; O, 21.99 | Bown liquid / white crystals after lyophilisation, hygroscopic |

Figure 11

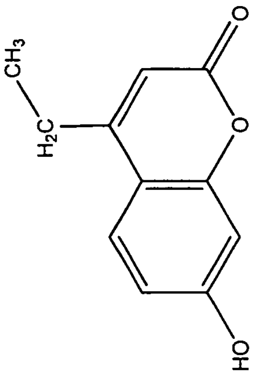

Chemical Formula: $C_{11}H_{10}O_3$
Exact Mass: 190.06
Molecular Weight: 190.20
m/z: 190.06 (100.0%), 191.07 (12.1%), 192.07 (1.3%)
Elemental Analysis: C, 69.46; H, 5.30; O, 25.24

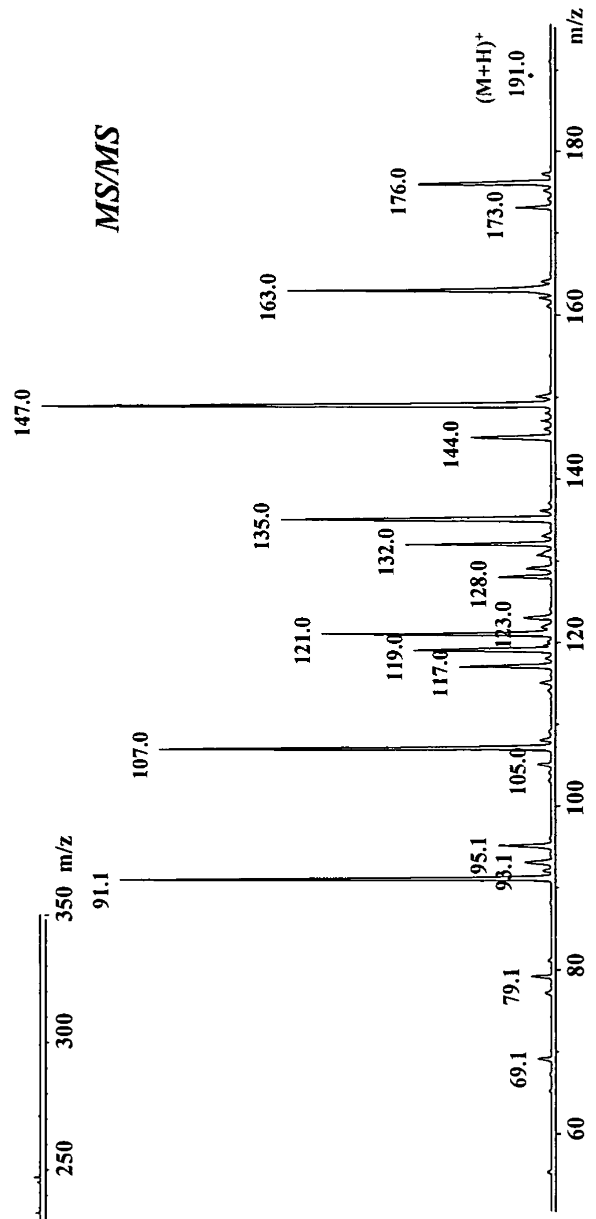


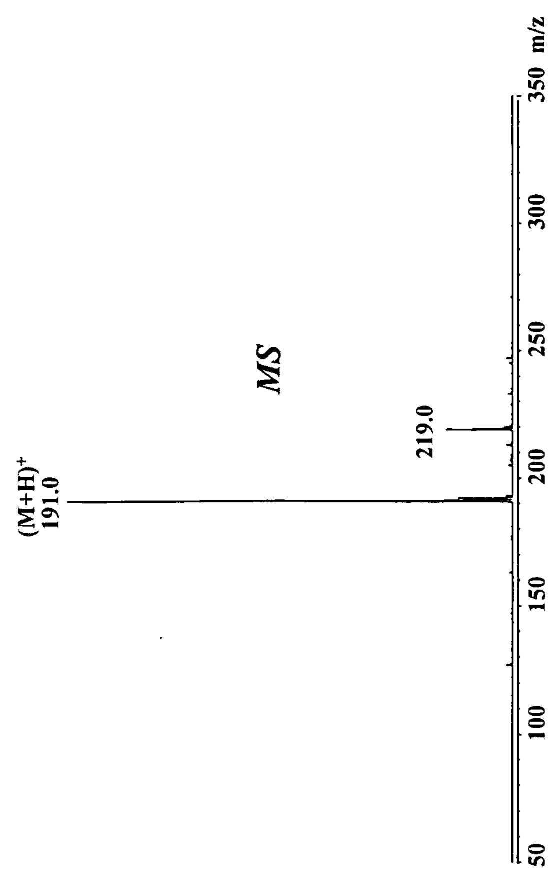


Figure 12

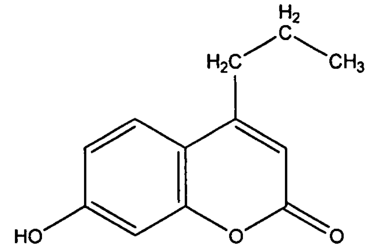

Chemical Formula: $C_{12}H_{12}O_3$
Exact Mass: 204.08
Molecular Weight: 204.22
m/z: 204.08 (100.0%), 205.08 (13.2%)
Elemental Analysis: C, 70.57; H, 5.92; O, 23.50

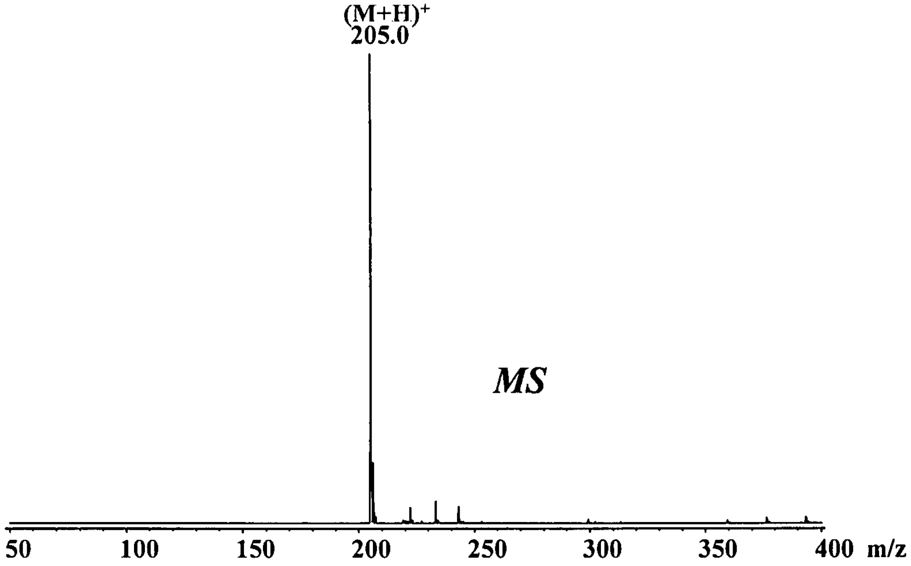


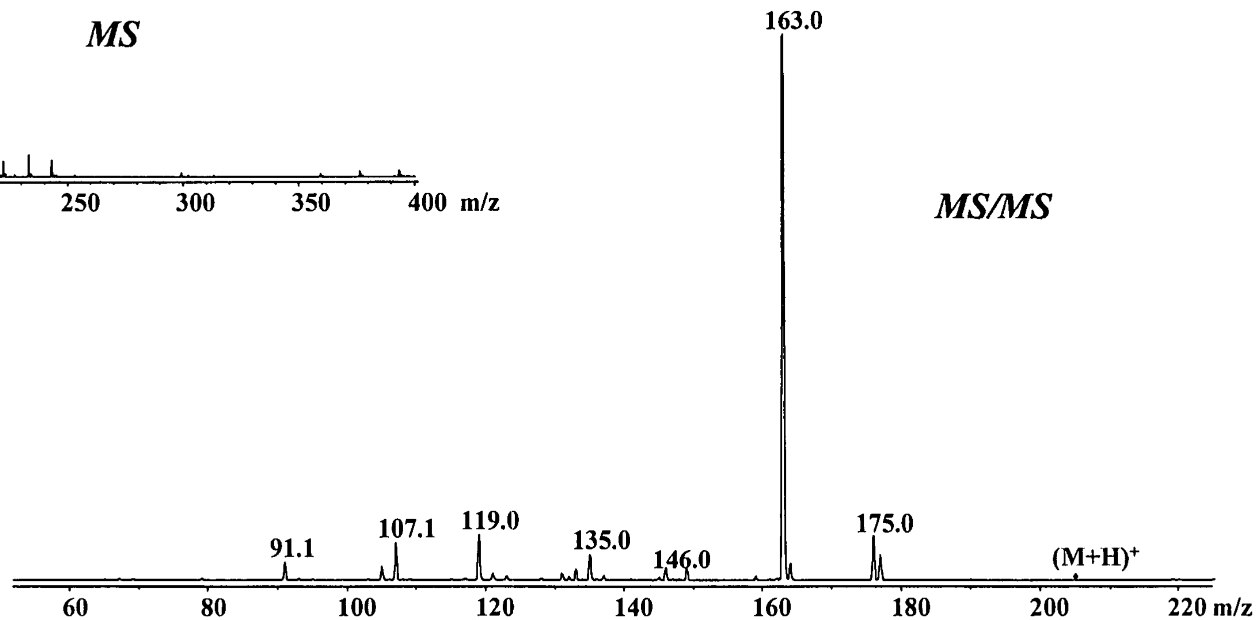


Figure 13

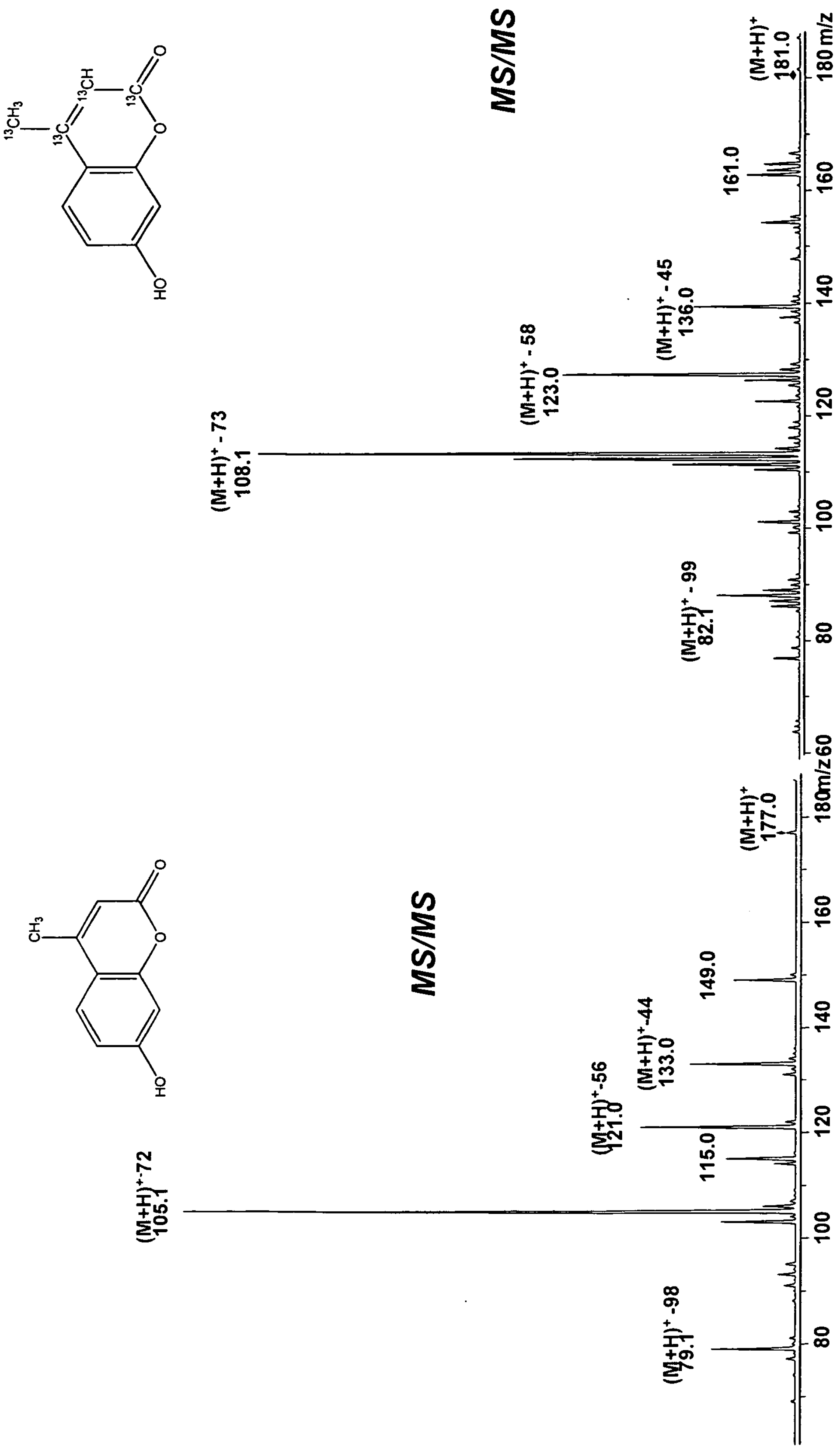
MS/MS
(M+H)+ 181.0
161.0
(M+H)+ - 45 136.0
(M+H)+ - 58 123.0
(M+H)+ - 73 108.1
(M+H)+ - 99 82.1
60 80 100 120 140 160 180 m/z
MS/MS
(M+H)+ 177.0
149.0
(M+H)+-44 133.0
(M+H)+-56 121.0
115.0
(M+H)+-72 105.1
(M+H)+ -98 79.1
80 100 120 140 160 180 m/z

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012027612 A1
- US 20110118132 A1


### Non-patent literature cited in the description


- **GERBER, S. A. et al.** *Anal. Chem.,* 2001, vol. 73, 1651-1657
- **WOLFE, B. J. et al.** *Anal. Chem.,* 2011, vol. 83, 1152-1156